Europäisches Patentamt
European Patent Office
Office européen des brevets

(19)

(11) Veröffentlichungsnummer: **0 060 223**
**A2**

(12)

# EUROPÄISCHE PATENTANMELDUNG

(21) Anmeldenummer: 82810099.0

(22) Anmeldetag: 04.03.82

(51) Int. Cl.³: C 07 D 249/08
C 07 D 233/56, C 07 D 401/06
C 07 D 409/06, A 01 N 43/64
A 01 N 43/50

(30) Priorität: 10.03.81 CH 1623/81
10.11.81 CH 7210/81
25.01.82 CH 435/82

(43) Veröffentlichungstag der Anmeldung:
15.09.82 Patentblatt 82/37

(84) Benannte Vertragsstaaten:
AT BE CH DE FR GB IT LI LU NL SE

(71) Anmelder: CIBA-GEIGY AG
Patentabteilung Postfach
CH-4002 Basel(CH)

(72) Erfinder: Kunz, Walter, Dr.
Im Goldbrunnen 55
CH-4104 Oberwil(CH)

(72) Erfinder: Nyfeler, Robert, Dr.
Bärenfelserstrasse 8
CH-4057 Basel(CH)

(72) Erfinder: Meyer, Alfred, Dr.
Bristenweg 6
CH-4054 Basel(CH)

(72) Erfinder: Frick, Willy, Dr.
Alter Kirchweg 15
CH-4148 Pfeffingen(CH)

(72) Erfinder: Maier, Ludwig, Dr.
Im Lee 28
CH-4144 Arlesheim(CH)

(72) Erfinder: Sturm, Elmar, Dr.
Klusstrasse 66
CH-4147 Aesch(CH)

(54) **Mikrobizide Mittel.**

(57) Es werden neue 1-Aryl-2-azolyl-ethen-derivate der allgemeinen Formel I beschrieben,

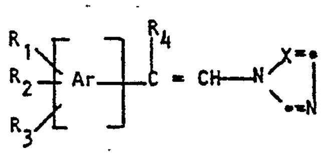

(I)

worin X für $-CH=$ oder $-N=$ steht;

Ar für Phenyl, Biphenyl-4-yl, α-Naphthyl, β-Naphthyl oder 4-Phenoxyphenyl steht;

$R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten;

$R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, 2-Pyridyl, 3-Pyridyl, 4-Pyridyl, 2-Thienyl oder eine durch $C_3$-$C_8$-Cycloalkyl, Phenoxy, Benzyloxy, $C_1$-$C_6$-Alkoxy, $-COO-C_1$-$C_4$-Alkyl, Cyano oder Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;

unter Einschluss der Säureadditionssalze, quaternären Azoliumsalze und Metallkomplexe.

Es werden ferner Methoden zur Herstellung dieser Produkte offenbart sowie agrochemische Schädlingsbekämpfungsmittel, die als Wirkstoff eine dieser Verbindungen enthalten. Ferner wird ein Verfahren zur Bekämpfung phytopathogener Mikroorganismen mit Hilfe dieser Substanzen beschrieben.

EP 0 060 223 A2

CIBA-GEIGY AG                    5-13646/1+2/+

Basel (Schweiz)

Mikrobizide Mittel

Die vorliegende Erfindung betrifft neue, substituierte 1-Aryl-2-azolyl-ethen-derivate sowie deren Säureadditionssalze, quaternäre Azoliumsalze und Metallkomplexe. Die Erfindung betrifft ferner die Herstellung dieser Substanzen sowie mikrobizide Mittel, die als Wirkstoff mindestens eine dieser Verbindungen enthalten. Die Erfindung betrifft auch die Herstellung der genannten Mittel und die Verwendung der Wirkstoffe oder der Mittel zur Bekämpfung von schädlichen Mikroorganismen. Sie betrifft darüber hinaus neue 1-Aryl-2-azolyl-ethan-derivate als wertvolle, mikrobizid aktive Zwischenprodukte für die Synthese obiger Wirksubstanzen.

Es werden hierin Verbindungen der allgemeinen Formel I

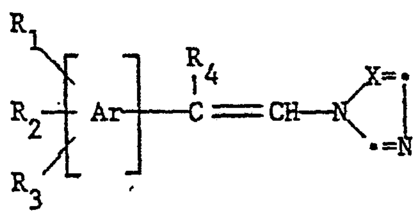

(I)

umfasst,

worin X für -CH= oder -N= steht;

Ar für Phenyl, Biphenyl-4-yl, α-Naphthyl, β-Naphthyl oder 4-Phenoxyphenyl steht;

$R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten;

$R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, Phenyl, 2-Pyridyl, 3-Pyridyl, 4-Pyridyl, 2-Thienyl oder eine durch $C_3$-$C_8$-Cycloalkyl, Phenoxy, Benzyloxy, $C_1$-$C_6$-Alkoxy, $-COO$-$C_1$-$C_4$-Alkyl, Cyano oder

Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;
unter Einschluss der Säureadditionssalze, quaternären Azoliumsalze und Metallkomplexe.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl usw. sowie ihre Isomeren, wie z.B. Isopropyl, Isobutyl, tert.-Butyl, Isopentyl usw.. Haloalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, $CHBr_2$ usw., insbesondere $CF_3$. Unter Halogen soll hier und im folgenden Fluor, Chlor, Brom oder Jod, vorzugsweise Fluor, Chlor oder Brom, verstanden werden. Alkenyl bedeutet z.B. Propenyl-(1), Allyl, Butenyl-(1), Butenyl-(2) oder Butenyl-(3).

Die vorliegende Erfindung betrifft somit die freien organischen Moleküle der Formel I, deren Säureadditionssalze, quaternäre Azoliumsalze. Dabei sind ganz besonders diejenigen Verbindungen im Umfang der Formel I bevorzugt, bei denen der Substituent $R_4$ für eine Niederalkyl- oder Cycloalkylgruppe steht.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Glykolsäure, Thiocyansäure, Milchsäure, Bernsteinsäure, Zitronensäure,

Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Zirkonium, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan, Zinn und Zirkonium sind bevorzugt.

Die Verbindungen der Formel I sind bei Raumtemperatur stabile Oele, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I bevorzugt. Die erfindungsgemässen Wirkstoffe der Formel I zeichnen sich in weiten Anwendungskonzentrationen durch sehr gute fungizide Aktivität und problemlose Anwendung ab.

Aufgrund ihrer ausgeprägten wuchsregulierenden und/oder mikrobiziden Wirkung sind diejenigen Wirksubstanzen der Formel I bevorzugt, die folgende Substituententypen oder Kombinationen dieser Substituenten untereinander aufweisen:

Bei Ar: a) Phenyl, Biphenyl-4-yl, α-Naphthyl, β-Naphthyl.

b) Phenyl, Biphenyl-4-yl, α-Naphthyl.

c) Phenyl, Biphenyl-4-yl.

d) Phenyl.

Bei $R_1$, $R_2$, $R_3$ unabhängig voneinander:

a) Wasserstoff, Halogen, Cyano, Nitro, $C_1$-$C_4$-Alkyl, $C_1$-$C_4$-Alkoxy, $C_1$-$C_3$ Haloalkyl.

b) Wasserstoff, Fluor, Chlor, Brom, Nitro, $C_1$-$C_3$-Alkyl, $C_1$-$C_3$-Alkoxy, Trifluormethyl.

c) Wasserstoff, Fluor, Chlor, Brom, Methyl, Ethyl, Methoxy, Ethoxy, Trifluormethyl.

d) Wasserstoff, Fluor, Chlor, Brom, Methyl, Methoxy, Trifluormethyl.

e) Wasserstoff, Fluor, Chlor, Brom, Methyl, Trifluormethyl.

f) $R_1$ = H, 2-F, 2-Br; 2-Cl, 2-$CH_3$. 2-$CF_3$
$R_2$ = H, 4-F, 4-Br, 4-Cl, 4-$CH_3$, 4-$CF_3$
$R_3$ = H, 6-F, 6-Br, 6-Cl, 6-$CH_3$, 6-$CF_3$.

Bei X: a) -CH=, -N=

b) -N=

Bei $R_4$: a) $C_1$-$C_{10}$-Alkyl, $C_3$-$C_8$-Cycloalkyl, Phenyl, $C_2$-$C_5$-Alkenyl, 2-Pyridyl, 3-Pyridyl, 4-Pyridyl, 2-Thienyl oder durch $C_3$-$C_6$-Cycloalkyl oder durch Phenyl substituiertes $C_1$-$C_4$-Alkyl, wobei jeder Phenylanteil unsubstituiert oder durch Fluor, Chlor, Brom, Methyl, Methoxy oder Trifluormethyl substituiert ist.

b) $C_1$-$C_6$-Alkyl, $C_3$-$C_6$-Cycloalkyl, Phenyl, oder durch $C_3$-$C_6$-Cycloalkyl oder durch Phenyl substituiertes $C_1$-$C_4$-Alkyl, wobei jeder Phenylanteil unsubstituiert oder durch Fluor, Chlor, Methyl, Methoxy oder Trifluormethyl substituiert ist.

c) $C_1$-$C_4$-Alkyl, $C_3$-$C_6$-Cycloalkyl, Phenyl oder durch $C_3$-$C_6$-Cycloalkyl oder durch Phenyl substituiertes $C_1$-$C_4$-Alkyl, wobei jeder Phenylanteil unsubstituiert oder durch Fluor, Chlor oder Methyl substituiert ist.

d) Methyl, Ethyl, n-Propyl, iso-Propyl, sek.-Propyl, n-Butyl, tert.-Butyl, sek.Butyl, iso-Butyl, Cyclopropyl, Cyclopentyl, Cyclohexyl, Phenyl, 2-Chlorphenyl, 2,4-Dichlorphenyl.

e) eine durch Phenoxy, Benzyloxy, $C_1$-$C_6$-Alkoxy, -COO-$C_1$-$C_4$-Alkyl, oder Cyano substituierte $C_1$-$C_{10}$-Alkylgruppe, wobei jeder Phenylanteil unsubstituiert oder durch Halogen, $C_1$-$C_4$-Alkyl, Methoxy oder Trifluormethyl substituiert ist.

f) eine durch Phenoxy, Benzyloxy oder $C_1$-$C_4$-Alkoxy substituierte $C_1$-$C_{10}$-Alkylgruppe, wobei jeder Phenylanteil durch Fluor, Chlor, Brom, Methyl, Ethyl, Methoxy oder Trifluormethyl substituiert ist.

Beispiele für besonders vorteilhafte Repräsentanten des Molekülfragments

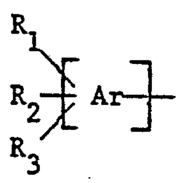

sind:

x)

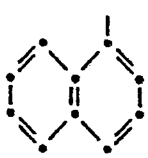

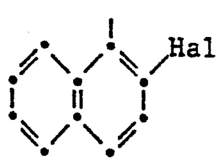

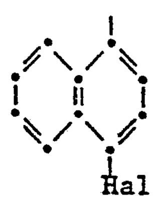

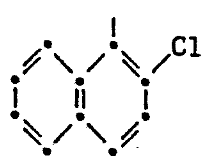

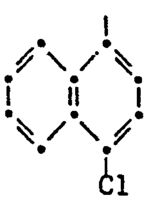

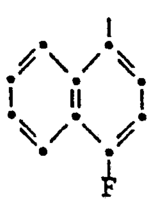

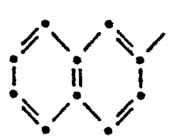

y)

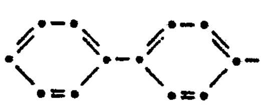

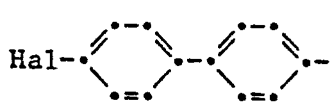

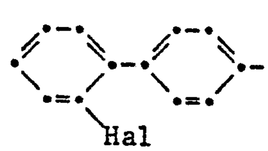

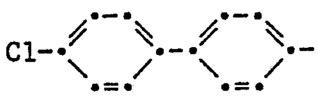 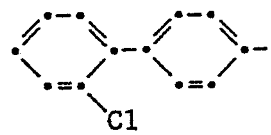

z) 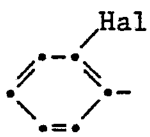 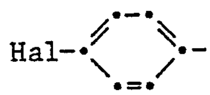 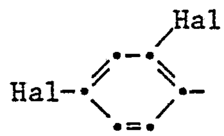 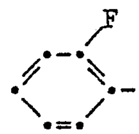

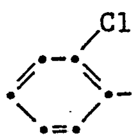 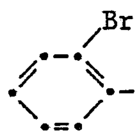 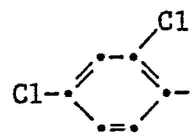 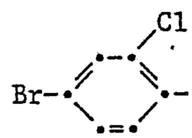

z') 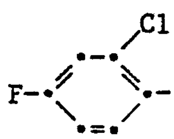 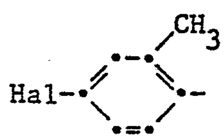

Besonders bevorzugt sind Verbindungen, die die Gruppe z) oder z') enthalten.

Es ergeben sich somit z.B. die nachfolgenden bevorzugten Gruppen a bis e:

a) Verbindungen der Formel I, worin Ar für Phenyl, Biphenyl-4-yl, α-Naphthyl oder β-Naphthyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; X für -CH= oder -N= steht; und $R_4$ $C_1$-$C_{10}$-Alkyl, $C_3$-$C_8$-Cycloalkyl, Phenyl, $C_2$-$C_5$-Alkenyl, 2-Pyridyl, 3-Pyridyl, 4-Pyridyl, 2-Thienyl oder eine durch $C_3$-$C_6$-Cycloalkyl oder durch Phenyl substituierte $C_1$-$C_5$-Alkylgruppe bedeutet, worin jeder Phenylanteil unsubstituiert oder durch Fluor, Chlor, Brom, Methyl, Methoxy oder Trifluormethyl substituiert ist.

b) Verbindungen der Formel I, worin Ar für Phenyl, Biphenyl-4-yl oder α-Naphthyl steht; $R_1$, $R_2$, $R_3$ unabhängig voneinander für Wasserstoff, Fluor, Chlor, Brom, Nitro, $C_1$-$C_3$-Alkyl, $C_1$-$C_3$-Alkoxy oder Trifluor-

methyl stehen; X für -N= steht; und $R_4$ $C_1$-$C_6$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor, Methyl, Methoxy oder Trifluormethyl substituiert ist.

c) Verbindungen der Formel I, worin Ar für Phenyl oder Biphenyl-4-yl steht; $R_1$, $R_2$, $R_3$ unabhängig voneinander Wasserstoff, Fluor, Chlor, Brom, Methyl, Ethyl, Methoxy oder Trifluormethyl bedeutet; X für -N= steht; und $R_4$ $C_1$-$C_4$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor oder Methyl substituiert ist.

d) Verbindungen der Formel I, worin das Molekülfragment

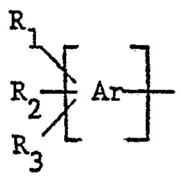

für α-Naphthyl, 2-Halo-α-naphthyl, 4-Halo-α-naphthyl, β-Naphthyl, Biphenyl-4-yl, 4'-Halobiphenyl-4-yl, 2'-Halobiphenyl-4-yl, 2-Halophenyl, 4-Halophenyl oder 2,4-Dihalophenyl steht; und $R_4$ $C_1$-$C_4$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor oder Methyl substituiert ist.

e) Verbindungen der Formel I, worin Ar für Phenyl steht; $R_1$ Wasserstoff, 2-Fluor, 2-Chlor, 2-Brom, 2-Methyl oder 2-Trifluormethyl; $R_2$ Wasserstoff, 4-Fluor, 4-Chlor, 4-Brom, 4-Methyl oder 4-Trifluormethyl, $R_3$ Wasserstoff, 6-Fluor, 6-Chlor, 6-Brom, 6-Methyl oder 6-Trifluormethyl bedeuten; und $R_4$ für Methyl, Ethyl, n-Propyl, iso-Propyl, sek.-Propyl, n-Butyl, sek.-Butyl, tert.-Butyl, iso-Butyl, Cyclopropyl, Cyclopentyl, Cyclohexyl, Phenyl, 2-Chlorphenyl oder 2,4-Dichlorphenyl steht.

Besonders bevorzugt sind Verbindungen der Gruppe d, insbesondere der Gruppe e.

Ferner sind folgende Untergruppen f, g und h interessant:

f) Verbindungen der Formel I, worin das Molekülfragment

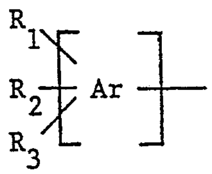

einen gegebenenfalls durch $C_1$-$C_4$-Alkyl, $C_1$-$C_4$-Alkoxy, Cyano, Nitro, Trifluormethyl oder ein bis drei Halogenatome substituierten Phenylrest bedeutet, X für -N= steht und $R_4$ für $C_1$-$C_{10}$-Alkyl, $C_2$-$C_5$-Alkenyl, $C_3$-$C_8$-Cycloalkyl, gegebenenfalls durch $C_3$-$C_8$-Cycloalkyl oder Phenyl substituiertes $C_1$-$C_4$-Alkyl steht, wobei der Phenylsubstituent seinerseits gegebenenfalls durch Halogen, $C_1$-$C_4$-Alkyl oder $C_1$-$C_4$-Alkxoy substituiert sein kann.

g) Verbindungen der Formel I, worin X für -CH= oder -N= steht; Ar für Phenyl, Biphenyl-4-yl, α-Naphthyl, β-Naphthyl oder 4-Phenoxyphenyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; $R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, Phenyl, 2-Pyridyl, 3-Pyridyl, 4-Pyridyl, 2-Thienyl oder eine durch $C_3$-$C_8$-Cycloalkyl oder Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;

unter Einschluss der Säureadditionssalze, quaternären Azoliumsalze und Metallkomplexe.

h) Verbindungen der Formel I, worin X für -CH= oder -N= steht; Ar für Phenyl, Biphenyl-4-yl, α-Naphthyl, β-Naphthyl oder 4-Phenoxyphenyl steht; $R_1$, $R_2$, $R_3$ unabhängig voneinander Wasserstoff,

Halogen, Cyano, Nitro, $C_1$-$C_4$-Alkyl, $C_1$-$C_4$-Alkoxy oder $C_1$-$C_3$-Haloalkyl bedeuten; $R_4$ eine durch Phenoxy, Benzyloxy, $C_1$-$C_6$-Alkoxy, -COO-$C_1$-$C_4$-Alkyl oder Cyano substituierte $C_1$-$C_{10}$-Alkylgruppe bedeutet, wobei jeder Phenylanteil unsubstituiert oder durch Halogen, $C_1$-$C_4$-Alkyl, Methoxy, Nitro oder Trifluormethyl substituiert ist; unter Einschluss der Säureadditionssalze, quaternären Azoliumsalze und Metallkomplexe.

Als Einzelsubstanzen sind folgende Verbindungen besonders hervorzuheben:

α-Ethyl-β-(1H-1,2,4-triazol-1'-yl)-2,4-dichlorstyrol,
α-(n)-Propyl-β-(1H-1,2,4-triazol-1'-yl)-2,4-dichlorstyrol,
α-iso-Propyl-β-(1H-1,2,4-traizol-1'-yl)-2,4-dichlorstyrol,
α-(n)-Butyl-β-(1H-1,2,4-triazol-1'-yl)-2,4-dichlorstyrol,
α-(n)-Pentyl-β-(1H-1,2,4-triazol-1'-yl)-2,4-dichlorstyrol,
α-sek.-Butyl-β-(1H-1,2,4-triazol-1'-yl)-2,4-dichlorstyrol,
α-Ethyl-β-(1H-1,2,4-triazol-1'-yl)-2-chlor-4-bromstyrol,
α-(n)-Propyl-β-(1H-1,2,4-triazol-1'-yl)-2-chlor-4-bromstyrol,
α-iso-Propyl-β-(1H-1,2,4-triazol-1'-yl)-2-chlor-4-bromstyrol,
α-(n)-Butyl-β-(1H-1,2,4-triazol-1'-yl)-2-chlor-4-bromstyrol,
α-sek.-Butyl-β-(1H-1,2,4-triazol-1'-yl)-2-chlor-4-bromstyrol,
α-n-Pentyl-β-(1H-1,2,4-triazol-1'-yl)-2-chlor-4-bromstyrol,
α-Cyclopropyl-β-(1H-1,2,4-triazol-1'-yl)-2,4-dichlorstyrol,
α-Cyclopropyl-β-(1H-1,2,4-triazol-1'-yl)-2-chlor-4-bromstyrol,
α-Cyclopentyl-β-(1H-1,2,4-triazol-1'-yl)-2,4-dichlorstyrol,
α-Cyclopentyl-β-(1H-1,2,4-triazol-1'-yl)-2-chlor-4-bromstyrol,
α-(n)-Propyl-β-(1H-1,2,4-triazol-1'-yl)-4-chlorstyrol,
α-(n)-Propyl-β-(1H-1,2,4-triazol-1'-yl)-2-chlorstyrol,
α-Cyclopropyl-β-1H-1,2,4-triazol-1'-yl)-4-chlorstyrol,
α-(n)-Propyl-β-(1H-1,2,4-triazol-1'-yl)-2-chlor-4-fluorstyrol,
α-Cyclohexyl-β-(1H-1,2,4-triazol-1'-yl)-4-chlorstyrol,
α-(n-)-Propyl-β-(1H-imidazol-1'-yl)-2,4-dichlorstyrol,
α-(4-Fluorphenoxymethyl)-β-(1H-1,2,4-triazol-1'-yl)-2,4-dichlorstyrol,

α-(4-tert.-Butylphenoxymethyl)-β-(1H-1,2,4-triazol-1'-yl)-2,4-dichlorstyrol,
α-(4-Chlorphenoxymethyl)-β-(1H-1,2,4-triazol-1'-yl)-2,4-dichlorstyrol,
α-(Phenoxymethyl)-β-(1H-1,2,4-triazol-1'-yl)-2,4-dichlorstyrol,
α-(2,4-Dichlorphenoxymethyl)-β-(1H-1,2,4-triazol-1'-yl)-4-methoxystyrol.

Die Verbindungen der Formel I können nach einer ganzen Reihe von Reaktionsvarianten i bis iv, wie anschliessend in einem Reaktionsschema skizziert und darauffolgend im einzelnen aufgeführt, hergestellt werden. In den Formeln I, II, III, IV, V, VI, VII, VII, IX, X, XI, XII, XIII, XIV und XV haben die Substituenten $R_1$, $R_2$, $R_3$, $R_4$, Ar und X die unter Formel I angegebenen Bedeutungen. $R_5$ steht für Phenyl oder $C_1$-$C_4$-Alkyl. $R_6$ für Phenyl oder gegebenenfalls durch Hydroxyl substituiertes $C_1$-$C_4$-Alkyl. A steht stellvertretend für eine der üblichen Abgangsgruppen, nämlich für Chlor, Brom, Jod oder die Gruppen $-OCO-R_7$ oder $-OSO_2-R_7$, wobei $R_7$ $C_1$-$C_3$-Alkyl, $C_1$-$C_3$-Haloalkyl oder gegebenenfalls durch Halogen, Methyl, Nitro, Trifluormethyl oder Methoxy substituiertes Phenyl bedeutet, vorzugsweise jedoch für Chlor oder Brom steht. Der Ausdruck Hal steht für Halogen, vorzugsweise für Fluor, Chlor oder Brom. M repräsentiert ein Alkalimetall, insbesondere Natrium oder Kalium.

REAKTIONS-SCHEMA

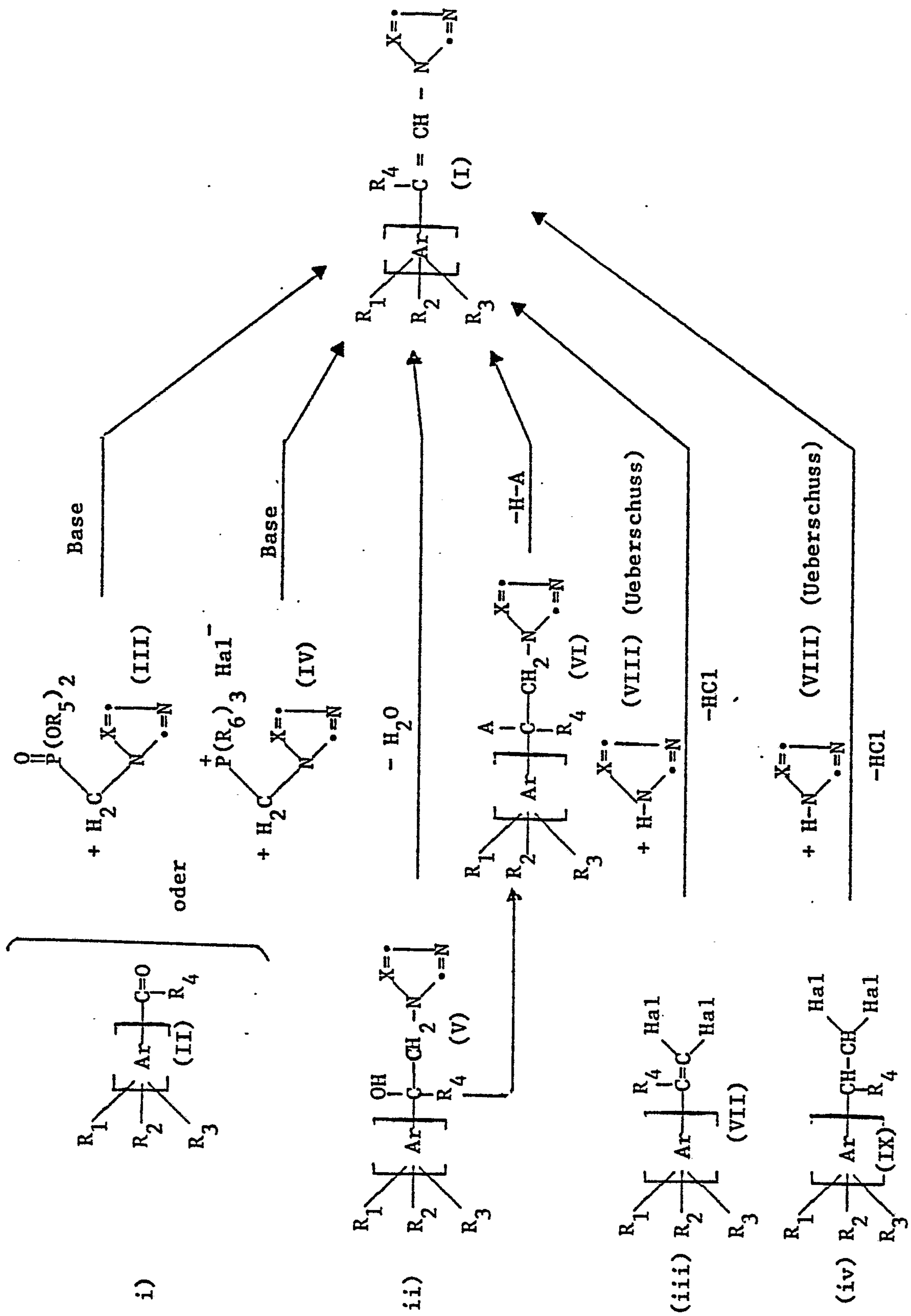


i)
ii)
(iii)
(iv)

Zur Herstellung der Verbindungen der Formel I kann man im einzelnen wie folgt vorgehen:

i) 1-Aryl-2-azolyl-ethen-derivate der Formel I können hergestellt werden, indem man ein Arylketon der Formel II

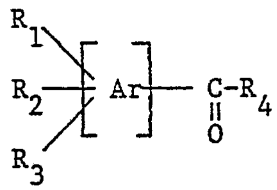

(II),

worin Ar, $R_1$, $R_2$, $R_3$ und $R_4$ die unter Formel I angegebenen Bedeutungen haben, durch Umsetzung mit einem 1H-Azol-1-yl-methylphosphonat der Formel III

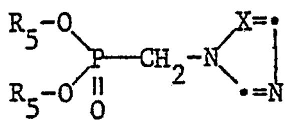

(III),

worin die Reste $R_5$ unabhängig voneinander Phenyl oder $C_1$-$C_4$-Alkyl bedeuten und X für -CH= oder -N= steht, oder mit einem Phosphoniumsalz der Formel IV

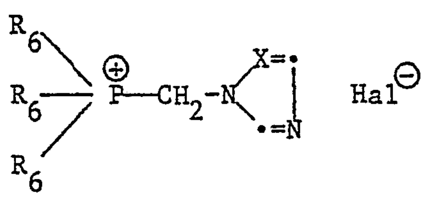

(IV),

worin die Reste $R_6$ unabhängig voneinander Phenyl oder gegebenenfalls durch Hydroxyl substituiertes $C_1$-$C_4$-Alkyl bedeuten, X für -CH= oder -N= steht, und Hal für Chlor, Brom steht, in einem reaktionsinerten Lösungsmittel in Gegenwart einer starken Base umsetzt.

Die Ueberführung der Arylketone der Formel II in die 1-Aryl-2-azolyl-ethen-derivate der Formel I kann somit nach der Verfahrensvariante entweder unter Verwendung der Phosphonate der Formel III oder der Phosphoniumsalze der Formel IV erfolgen.

Die Reaktionsvariante, die als Reaktionspartner die Phosphonate der Formel III verwendet, wird vorteilhafterweise so geführt, dass man

das Phosphonat zunächst mit einer starken Base und danach mit dem Phenylketon der Formel II versetzt.

Als Lösungsmittel bei dieser Eintopfreaktion können inerte, polare, aprotische Lösungsmittel verwendet werden. Solche Lösungsmittel sind Ether wie Diethylether, Tetrahydrofuran, Dioxan, Dimethoxyethan oder Diethylenglykol-dimethylether; Säureamide wie Dimethylformamid, 2-Pyrrolidinon oder Hexamethylphosphorsäuretriamid; und Sulfoxide wie Dimethylsulfoxid.

Bevorzugt sind Lösungsmittel, deren Siedepunkt über +60°C liegt, also z.B. Tetrahydrofuran, Dioxan, Dimethoxyethan, Dimethylformamid oder Dimethylsulfoxid.

Als starke Basen können verwendet werden: metallorganische Verbindungen wie Methyllithium, Propyllithium, Butyllithium, Phenyllithium, oder Triphenylmethylnatrium; Alkoholate wie Natriummethylat, Natriumethylat, Kaliumethylat oder Kalium-tert.-butylat; Metallhydride wie Lithiumhydrid, Natriumhydrid oder Calciumhydrid; und Alkaliamide wie Natriumamid oder Lithiumdiisopropylamid.

Als bevorzugte Basen sind die Metallhydride, die metallorganischen Verbindungen und die Alkoholate zu verstehen.

Da es sich sowohl bei der Umsetzung des Phosphonats III mit der starken Base zum entsprechenden Salz, als auch bei der weiteren Reaktion mit dem Keton II um exotherme Vorgänge handelt, kühlt man das Reaktionsgefäss jeweils vor dem Zusetzen des Reagenzes ab und erwärmt dann anschliessend zur Vervollständigung der Umsetzung. Geeignete Temperaturintervalle sind für die erste Stufe -40° bis +40°C, insbesondere -20° bis +40°C und für die zweite Stufe -20° bis +80°C, insbesondere 0° bis +60°C.

Das entstandene 1-Aryl-2-azolyl-ethen-derivat der Formel I wird isoliert, indem man das Reaktionsgemisch mit Wasser versetzt, einem mit Wasser nicht mischbaren Lösungsmittel extrahiert und die organischen Phasen eindampft.

Die Reaktionsvariante, die als Reaktionspartner die Phosphoniumsalze der Formel IV verwendet, wird vorteilhafterweise so geführt, dass man das Phosphoniumsalz zunächst mit einer starken Base und danach mit dem Phenylketon der Formel II versetzt.

Es hat sich auch hier als günstig erwiesen, wenn man die Base im Ueberschuss zusetzt. Ueblicherweise beträgt der Ueberschuss weniger als das Doppelte der zur Reaktion benötigten molaren Menge, also zwischen 0,01 und 1 Aequivalenten.

Als Lösungsmittel bei dieser Eintopfreaktion können wiederum inerte, polare, aprotische Lösungsmittel verwendet werden. Solche Lösungsmittel sind Ether wie Diethylether, Tetrahydrofuran, Dioxan, Dimethoxyethan oder Diethylenglykol-dimethylether; Säureamide wie Dimethylformamid, 2-Pyrrolidinon oder Hexamethylphosphorsäuretriamid; und Sulfoxide wie Dimethylsulfoxid.

Bevorzugt sind Lösungsmittel, deren Siedepunkt über 80°C liegt, also z.B. Dioxan, Dimethoxyethan, Diethylenglykol-dimethylether, Dimethylformamid, 2-Pyrrolidinon oder Dimethylsulfoxid.

Als starke Basen können verwendet werden: metallorganische Verbindungen wie Methyllithium, Propyllithium, Butyllithium, Phenyllithium,

oder Triphenylmethylnatrium; Alkoholate wie Natriummethylat, Natriumethylat, Kaliumethylat oder Kalium-tert.-butylat; Metallhydride wie Lithiumhydrid, Natriumhydrid oder Calciumhydrid; und Alkaliamide wie Natriumamid, oder Lithiumdiisopropylamid.

Als bevorzugte Basen sind die Metallhydride und die metallorganischen Verbindungen zu verstehen.

Da es sich sowohl bei der Umsetzung des Phosphoniumsalzes mit der starken Base zum entsprechenden Ylid, als auch bei der weiteren Reaktion mit dem Keton II um exotherme Vorgänge handelt, kühlt man das Reaktionsgefäss jeweils vor dem Zusetzen des Reagenses ab und erwärmt dann anschliessend zur Vervollständigung der Umsetzung. Geeignete Temperaturintervalle sind für die erste Stufe -40° bis +80°C, insbesondere -20° bis +40°C und für die zweite Stufe -20° bis +110°C, insbesondere 0° bis +60°C.

Das entstandene 1-Aryl-2-azolyl-ethen-derivat der Formel I wird isoliert, indem man das Reaktionsgemisch mit Diethylether verdünnt, das ausgefallene Phosphinoxid abfiltriert und das Filtrat eindampft. Um am Phosphinoxid anhaftendes Produkt zu gewinnen, kann es notwendig sein, den Filterkuchen mehrmals mit Ether auszuwaschen.

In den Fällen, in denen man bei der Herstellungsvariante i von Salzen der Formeln III oder IV ausgeht, kann sich der Zusatz eines Kronenethers als sehr vorteilhaft erweisen. So ist beispielsweise der Einsatz von [18]-Krone-6 und [15]-Krone-5 sehr günstig.

Bei der Herstellungsvariante i entstehen üblicherweise Gemische von cis- und trans-Olefinen, in denen der cis-Olefinanteil im allgemeinen überwiegt. Die Bildung eines höheren trans-Olefin-anteils kann durch einen grösseren Ueberschuss am Ylid oder durch Zusatz von Lithiumsalzen (z.B. $LiClO_4$) erzielt werden. [Vgl. M. Schlosser, Chem. Ber. 103, 2841].

ii) 1-Aryl-2-azolyl-ethen-derivate der Formel I können ferner hergestellt werden, indem man entweder direkt aus einer Verbindung der Formel V

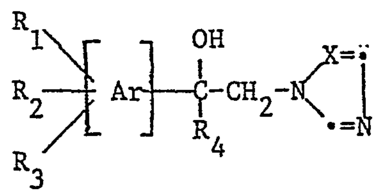

(V),

worin Ar, $R_1$, $R_2$, $R_3$, $R_4$ und X die unter Formel I angegebenen Bedeutungen haben, mit einem wasserentziehenden Mittel Wasser abspaltet; oder indem man vorteilhafterweise die Verbindung der Formel V durch Austausch der freien Hydroxylgruppe gegen eine der üblichen Abgangsgruppen A zuerst in eine Verbindung der Formel VI überführt

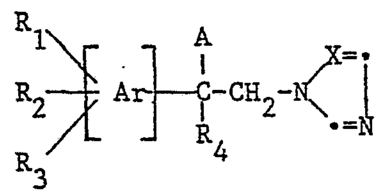

(VI)

und VI dann durch Abspaltung von H-A in die Endprodukte der Formel I verwandelt. Unter einer üblichen Angangsgruppe A sind im Rahmen vorliegender Erfindung die Substituenten Chlor, Brom, Jod sowie die Gruppen $-OCO-R_7$ und $-OSO_2-R_7$ zu verstehen, wobei $R_7$ für $C_1-C_3$-Alkyl, $C_1-C_3$-Haloalkyl oder gegebenenfalls durch Halogen, Methyl, Nitro, Trifluormethyl oder Methoxy substituiertes Phenyl steht, vorzugsweise jedoch für Chlor oder Brom.

Die Reaktion kann in vielen Fällen im sogenannten Eintopfverfahren durchgeführt werden, d.h. das Zwischenprodukt der Formel VI kann zwar, muss aber nicht, aus dem Reaktionsmedium isoliert werden.

Der Wasserentzug aus Verbindungen der Formel V erfolgt zweckmässigerweise in einem üblichen reaktionsinerten Lösungsmittel oder Lösungsmittelgemisch. Geeignete Lösungsmittel sind z.B. Alkohole, wie Niederalkanole (Methanol, Ethanol, n-Propanol, iso-Propanol, n-Butanol, Amylalkohol usw.); Ether und etherartige Verbindungen, wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Xylol, Petrolether; halogenierte Kohlenwasserstoffe wie Chlorbenzol, Methylenchlorid, Ethylenchlorid, Chloroform, Tetrachlorkohlenstoff, Tetrachlorethylen usw. Als wasserentziehende Mittel kommen z.B. starke Säuren, insbesondere konzentrierte oder verdünnte Mineralsäuren wie Phosphorsäure, Schwefelsäure oder Halogenwasserstoffsäuren (Chlorwasserstoff-, Bromwasserstoff-, Jodwasserstoff- oder Fluorwasserstoffsäure in Frage. Die Reaktion wird bei Temperaturen von 0° bis +180°C durchgeführt, üblicherweise bei erhöhter Temperatur. Als wasserentziehendes Mittel können auch Carbodiimide wie N,N'-Dicyclohexylcarbodiimid eingesetzt werden. Hier liegen die Reaktionstemperaturen bei 0° bis +150°C.

Der Austausch der freien Hydroxylgruppe in den Verbindungen der Formel V gegen eine Abgangsgruppe A wird bevorzugt in einem reaktionsinerten Lösungsmittel durchgeführt. Beispiele solcher Lösungsmittel sind: Aromatische und aliphatische Kohlenwasserstoffe wie Benzol, Toluol, Xylole, Petrolether, Ligroin oder Cyclohexan; halogenierte Kohlenwasserstoffe wie Chlorbenzol, Methylenchlorid, Ethylenchlorid, Chloroform, Tetrachlorkohlenstoff oder Tetrachlorethylen; Ether und etherartige Verbindungen wie Diethylether, Diisopropylether, T-Butylmethylether, Dimethoxyethan, Dioxan, Tetrahydrofuran oder Anisol; Ester wie Ethylacetat, Propylacetat oder Butylacetat; Nitrile wie Acetonitril; oder Verbindungen wie Dimethylsulfoxid, Dimethylformamid und Gemische solcher Lösungsmittel untereinander.

Die Einführung der Abgangsgruppe A erfolgt nach üblichen Methoden. Bedeutet A Chlor, so wird als Reagenz z.B. Phosphoroxychlorid, Phosphortrichlorid, Phosphorpentachlorid oder vorzugsweise Thionylchlorid eingesetzt. Man arbeitet im allgemeinen bei Temperaturen von 0° bis +120°C. Im Falle von A = Brom verwendet man bevorzugt Phosphortribromid oder Phosphorpentabromid und führt die Reaktion bei 0° bis +50°C durch. Steht A für eine der Gruppen $-OCO-R_7$ oder $-OSO_2-R_7$ so wird als Reagenz üblicherweise das entsprechende Säurehalogenid, insbesondere Säurechlorid, eingesetzt. Hierbei ist es zweckmässig, wenn

die Reaktion bei Temperaturen von -20° bis +50°C, vorzugsweise -10° bis +30°C, und in Gegenwart einer schwachen Base wie Pyridin oder Triethylamin durchgeführt wird.

Zur Abspaltung der hydrierten Abgangsgruppe (H-A) wird eine geeignete Base zugesetzt. Beispiele derartiger Basen sind: tertiäre Amine (Triethylamin, Ethyldiisopropylamin etc.); bicyclische Amine wie 1,4-Diazabicyclo[2.2.2]octan, 1,5-Diazabicyclo]3.4.0]-nonen-5 usw.; Dialkylaniline wie N,N-Dimethylanilin, N,N-Methylethylanilin usw.; heterocyclische Basen wie Pyridin, Collidin, Chinolin usw.; ferner anorganische Basen wie Natriumacetat, Natriumhydrogencarbonat, Alkali- und Erdalkalihydroxide [NaOH, KOH, $Ca(OH_2$, $Ba(OH)_2$]; Alkalialkoholate [Natrium- oder Kaliumethanolat, Kaliumtertiärbutylat] usw. Die Umsetzung erfolgt bei einem Temperaturbereich von 0° bis +120°C.

iii) Bei einer anderen Herstellungsvariante für Verbindungen der Formel I geht man von 1-Aryl-2,2-dichlor-ethen-derivaten der Formel VIII aus

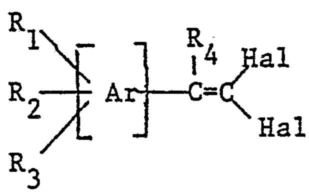

(VII),

worin Ar, $R_1$, $R_2$, $R_3$ und $R_4$ die unter Formel I angegebenen Bedeutungen haben, die Substituenten Hal unabhängig voneinander für Halogen, insbesondere für Fluor, Chlor oder Brom stehen, und setzt diese in der Schmelze bei +150° bis +350°C und gegebenenfalls unter Druck mit überschüssigem Azol der Formel VII um,

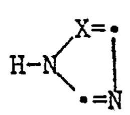

(VIII)

worin X für -CH= oder -N= steht. Man verwendet hierbei bis zu zehnfachen Ueberschuss an Azol VIII, bevorzugt jedoch seine dreifache Menge, bezogen auf die Verbindung der Formel VII.

iv) Verbindungen der Formel I lassen sich ferner aus Verbindungen der Formel IX herstellen

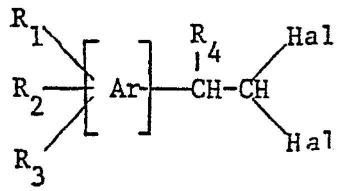 (IX),

worin Ar, $R_1$, $R_2$, $R_3$ und $R_4$ die unter Formel I angegebenen Bedeutungen haben, und die Substituenten Hal unabhängig voneinander für Halogen, insbesondere für Fluor, Chlor oder Brom stehen, indem man letztere in einem dipolaren aprotischen Lösungsmittel, gegebenenfalls in Gegenwart einer Base, bei +40° bis +180°C, vorzugsweise +80° bis +120°C, oder in der Schmelze bei +150° bis +350°C mit überschüssigem Azol der Formel VIII herstellt.

Die Base kann im Verlauf des Schmelzvorganges in das Reaktionsgemisch eingetragen werden. Falls erforderlich, wird unter Druck (bis 15 bar) gearbeitet. Als Basen kommen insbesondere anorganische Basen wie Alkali- und Erdalkalihydroxide (KOH, NaOH) in Frage. Geeignete aprotische Lösungsmittel sind beispielsweise Dimethylformamid, Dimethylsulfoxid, Hexamethylphosphorsäuretriamid und Nitrile wie Acetonitril.

Die Varianten iii und iv eignen sich insbesondere in den Fällen, in denen der Substituent $R_4$ einen aromatischen Rest darstellt.

Die Ausgangsverbindungen der Formel II sind zum Teil bekannt und im Handel erhältlich, oder sie können durch Friedel-Crafts-Acylierung aus den entsprechenden Carbonsäurehalogeniden und Aromaten leicht hergestellt werden.

Die 1H-Azol-1-yl-methylphosphonate der Formel III lassen sich dadurch herstellen, dass man ein 1-Halogenmethyl-1H-Azol der Formel X

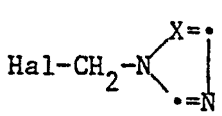 (X),

worin Hal für Chlor, Brom oder Jod steht, entweder mit einem sekundären Phosphit der Formel XI

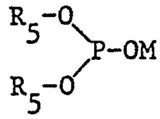 (XI),

worin die Reste $R_5$ unabhängig voneinander Phenyl oder $C_1$-$C_4$-Alkyl bedeuten und M für ein Alkalimetallatom steht, oder mit einem tertiären Phosphit der Formel XII umsetzt

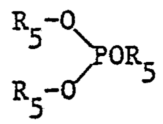 (XII),

worin $R_5$ die unter Formel (XI) angegebenen Bedeutungen hat.

Mit Vorteil wird das Verfahren zur Herstellung der Phosphonate der Formel III in einem inerten organischen Lösungsmittel durchgeführt. Solche Lösungsmittel sind aromatische Kohlenwasserstoffe wie Benzol, Toluol oder Xylol; Ether wie Diethylether, Tetrahydrofuran, Dioxan, Dimethoxyethan oder Diethylenglykol-dimethylether oder Nitrile wie Acetonitril. Verwendet man ein sekundäres Alkaliphosphit der Formel XI, ist es angebracht, ein polares Lösungsmittel wie Acetonitril, Dimethoxyethan oder Diethylenglykol-dimethylether einzusetzen. Wird zur Syntnese der Phosphonate ein tertiäres Phosphit der Formel VII eingesetzt, so kann dieses selbst häufig als Lösungsmittel dienen.

In jedem Fall jedoch empfiehlt es sich, die Reaktionsmischung zu erwärmen; bei der sekundär-Phosphitreaktion auf 50° bis 150°C, vorzugsweise auf +80° bis +120°C und bei der tertiär-Phosphitreaktion auf +100° bis +180°C, vorzugsweise auf +120° bis +160°C.

Bei den sekundären Alkaliphosphiten handelt es sich im allgemeinen um Natrium- oder Kaliumphosphite.

Die Phosphite der Formel III werden isoliert, indem man gegebenenfalls den entstandenen Niederschlag abtrennt, die Lösung eindampft und den Rückstand destilliert.

Die 1H-Azol-1-yl-methylphosphoniumsalze der allgemeinen Formel IV lassen sich herstellen, indem man ein 1-Halogenmethyl-1H-Azol der Formel X mit einem Phosphin der Formel XIII umsetzt

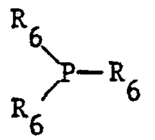

(XIII),

worin $R_6$ die unter Formel IV angegebenen Bedeutungen hat.

Mit Vorteil wird das Verfahren zur Herstellung der Phosphoniumsalze der Formel IV in einem inerten, organischen Lösungsmittel durchgeführt. Solche Lösungsmittel sind aromatische Kohlenwasserstoffe wie Benzol, Toluol oder Xylol; Ether wie Diethylether, Tetrahydrofuran, Dioxan, Dimethoxyethan oder Diethylenglykol-dimethylether; Säureamide wie Dimethylformamid oder 2-Pyrrolidinon oder Nitrile wie Acetonitril. Vorzugsweise werden polare Lösungsmittel wie Dimethylformamid, Acetonitril oder Dimethoxyethan verwendet.

Es empfiehlt sich, das Reaktionsgemisch auf +30° bis +120°C, vorzugsweise auf +50° bis +100°C, zu erwärmen.

Beim Abkühlen der Reaktionsmischung kristallisieren die Phosphoniumsalze meist in reiner Form aus, so dass sich ein Extraktions- oder Fällungsverfahren zur Isolation dieser Verbindungen im allgemeinen erübrigt. Fällt das Produkt nicht direkt an, so kann es durch einfaches Abdampfen des Lösungsmittels erhalten werden.

Einige der Phosphoniumsalze der Formel IV haben selbst auch fungizide Eigenschaften und sind, sofern sie neu sind, ein Teil der Erfindung.

Die 1-Halogenmethyl-1H-azole der Formel X

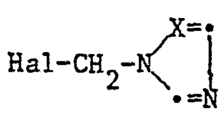

(X),

worin Hal für Chlor, Brom oder Jod steht, werden erhalten, indem man 1-Hydroxymethyl-1H-azol der Formel XIV

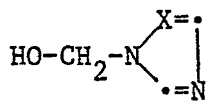 (XIV)

mit einem Halogenierungsmittel umsetzt und die entstandenen Hydrohalogenide der Formel XV

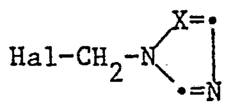 • HHal (XV),

worin Hal die unter Formel X angegebene Bedeutung hat, mit einer Base behandelt.

Als Basen eignen sich starke anorganische Basen, z.B. Hydroxide wie Natrium- und Kaliumhydroxid.

Als Halogenierungsmittel können Verwendung finden: Phosgen, Thionylchlorid, Thionylbromid, Phosphortrichlorid, Phosphortribromid, Phosphorpentachlorid, Phosphoroxychlorid oder Jodwasserstoffsäure. Vorzugsweise werden Thionylchlorid und Thionylbromid zur Halogenierung eingesetzt, da die entstehenden Nebenprodukte gasförmig sind, aus der Reaktionslösung entweichen und deshalb die Reaktion nicht beeinflussen. Die Jodverbindungen werden mit Vorteil aus bereits halogenierten Verbindungen erhalten, wenn man diese mit Jodwasserstoffsäure umsetzt. Eine weitere Methode zur Herstellung von Chloriden, Bromiden und Jodiden besteht in der Umsetzung von XIV mit den entsprechenden Trialkylsilyl-Halogeniden.

Die Halogenierungsreaktion wird in inerten Lösungsmitteln ausgeführt, wie z.B. Kohlenwasserstoffen wie Hexan, Cyclohexan, Benzol, Toluol oder Xylol oder Ethern wie Diethylether, Tetrahydrofuran, Dioxan oder Dimethoxyethan. Bei Verwendung von flüssigen Halogenierungsmitteln kann häufig ganz auf ein Lösungsmittel verzichtet werden. Die Reaktion wird dann in einem Ueberschuss des Reagenzes, z.B. Thionylchlorid chlorid oder -bromid, durchgeführt.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid. Ferner kann das Arbeiten unter erhöhtem Druck günstig auf die Ausbeute auswirken.

Die Ausgangsprodukte der Formel V sind grösstenteils bekannt oder werden analog zu beschriebenen Methoden hergestellt (vgl. GB-PS 2,064,520).

Verbindungen des Typs der Formel VII sind ebenfalls aus der EP-PS 0,004,315 bekannt oder können nach den dort beschriebenen Methoden hergestellt werden. Auch Verbindungen des Typs der Formel IX sind aus der Literatur bekannt oder lassen sich analog zu den beschriebenen Verfahren herstellen [vgl. JACS 67, 1591 (1945)]. Die Ausgangsverbindungen der Formeln VIII, XI, XII, XIII und XIV sind allgemein bekannt, sie stellen im allgemeinen Grundchemikalien dar und können nach an sich bekannten Methoden hergestellt werden. Sie sind grösstenteils im Handel erhältlich.

Die Substanzen der Formel VI sind neu, sie stellen besonders entwickelte Zwischenprodukte zur Herstellung der wertvollen Wirkstoffe der Formel I dar. Aufgrund ihrer strukturellen Beschaffenheit lassen sie sich leicht in die Verbindungen der Formel I überführen. Darüberhinaus zeigen Verbindungen der Formel VI wertvolle fungizide Aktivität gegenüber Schadpilzen aus den Familien Fungi imperfecti, Basidiomycetes oder Ascomycetes. Die Verbindungen der Formel VI sind einschliesslich ihres Herstellungsverfahrens ein Bestandteil der vorliegenden Erfindung. Sie enthalten in den meisten Fällen ein asymmetrisches C-Atom in Nachbarstellung zum Substituenten A und fallen im allgemeinen als Racemate an, die auf übliche Weise in die optischen Antipoden aufgetrennt werden können. Im allgemeinen zeigen die optisch reinen Isomeren eine unterschiedliche fungizide Wirkung.

Sofern sie zu neuen Produkten führen, sind die hierin beschriebenen Herstellungsvarianten i, ii, iii und iv, einschliesslich aller Teilschnitte, ein wichtiger Bestandteil dieser Erfindung.

Zwei zu den hierin beschriebenen Wirkstoffen der Formel I strukturell ähnliche Substanzen, nämlich die nachfolgenden Triazolderivate XX und XXX

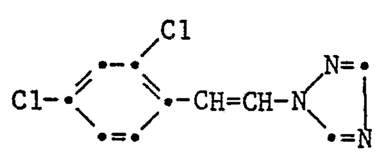

;

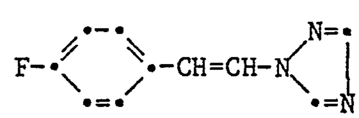

(XX) (XXX)

sind aus der DE-OS-2,833,194 als Synergisten für Insektidzide bekannt.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen

einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben. Eine ähnliche Aktivität zeigen auch die Zwischenprodukte der Formel VI.

Die Wirkstoffe der Formel I sind gegen die den folgenden Klassen angehörenden phytopathogenen Pilze wirksam: Fungi imperfecti (z.B. Botrytis, Helminthosporium, Fusarium Septoria, Cercospora und Alternaria); Basidiomyceten (z.B. die Gattungen Hemileia, Rhizocotonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkuturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst:

(Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das

Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emuslionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie

Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichelichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" BC Publishing Corp., Ringwood New Jersey, 1980
Sisely and Wood, "Encyclopedia of Surgace Active Agents", Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viksositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid. Druckangaben erfolgen in Millibar mb, oder Bar b.

Herstellungsbeispiele für Ausgangsprodukte:

Beispiel 1: Herstellung von

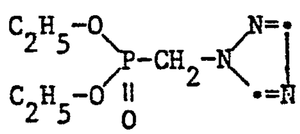

Diethyl-(1H-1,2,4-triazol-1-yl)-methylphosphonat

a) Zur Dispersion von 17,6 g (0,1 Mol) Kalium-diethylphosphit in 100 ml Toluol lässt man bei 90° 11,7 g (0,1 Mol) frisch hergestelltes 1-Chlormethyl-1H-1,2,4-triazol zutropfen. Nach Beendigung der exothermen Reaktion wird das ausgefallene Kaliumchlorid abgetrennt und das Filtrat eingedampft. Die Chromatographie des Rückstands mit Ethylacetat an Kieselgel ergibt 2,1 g(9,6%) gelbes Oel. Durch Kugelrohrdestillation dieses Oels erhält man Diethyl-(1H-1,2,4-triazol-1-yl)-methylphosphonat als farbloses Oel, Kp. 130°/0,11 mb.

$^{1}H$-NMR ($CDl_3$, TMS): $\delta$= 8,4 (s; 1H, Triazol 5-CH); 8,07 (s; 1H, Triazol 3-CH); 4,75 (d, $J_{PCH}$ = 13 Hz; 2H, P-$CH_2$); 4,25 (2q, J = Hz; 4H, $OCH_2$) und 1,5 (t, J = Hz; 6H, $CH_3$) ppm.

$^{31}P$-NMR ($CD_3OD$, $H_3PO_4$): $\delta$ = +17,12 ppm.

b) Eine Mischung aus 18,2 g (0,155 Mol) 1-Chlormethyl-1H-1,2,4-triazol, 27,2 g (0,17 Mol) Natrium-diethylphosphit und 200 ml Acetonitril wird für 2 h auf 60° erwärmt und anschliessend für 16 h unter Rückfluss erhitzt. Nach Abtrennen des Niederschlages wird die Lösung eingedampft und fraktioniert destilliert. Man erhält 19,1 g (56,5%) Diethyl-(1H-1,2,4-triazol-1-yl)-methylphosphonat als farbloses Oel. Kp. 120-129°/0,053 mb.

$C_7H_{14}N_3O_3P$ (219,2)

Ber.: C 38,36%; H 6,44%; N 19,17%;
Gef.: C 38,37%; H 6,60%; N 19,76%.

Beispiel 2: Herstellung von

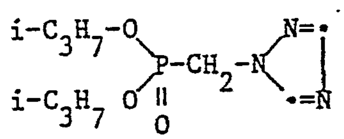

Di-i-propyl-(1H-1,2,4-triazol-1-yl)-methylphosphonat

Zur Dispersion von 18,8 g (0,1 Mol) Natrium-di-i-propylphosphit in 100 ml Toluol lässt man bei 90° 11,7 g (0,1 Mol) frisch hergestelltes 1-Chlormethyl-1H-1,2,4-triazol zutropfen. Nach Beendigung der exothermen Reaktion wird das ausgefallene Natriumchlorid abgetrennt und das Filtrat eingedampft. Die Chromatographie des Rückstands mit Ethylacetat an Kieselgel ergibt 2,9 g (11,7%) gelbes Oel. Durch Kugelrohrdestillation dieses Oels erhält man Di-i-propyl-(1H-1,2,4-triazol-1-yl)-methylphosphonat als farbloses Oel.

$^1$H-NMR ($CDCl_3$, TMS): $\delta$ = 8,4 (s; 1H, 5-CH); 7,93 (s; 1H, 3-CH); 4,6 (m, 2H, O-CH); 4,5 (d, $J_{PCH}$ = 13 Hz; 2H, P-$CH_2$) und 1,2 (α, J = 7Hz; 12H, $CH_3$) ppm.

Beispiel 3: Herstellung von

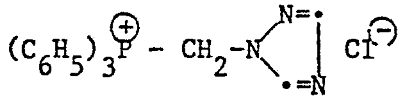

1H-1,2,4-Triazol-1-yl-methyl-triphenyl-phosphoniumchlorid

Eine Lösung von 40,6 g (0,345 Mol) 1-Chlormethyl-1H-1,2,4-triazol und 90,5 g (0,345 Mol) Triphenylphosphin in 250 ml Acetonitril wird für 16 h unter Rückfluss erhitzt. Beim Abkühlen der Lösung scheiden sich 91,7 g (70,0%) 1H-1,2,4-Triazol-1-yl-methyl-triphenyl-phosphoniumchlorid als farblose Kristalle ab, Fp. 287°.

$C_{21}H_{19}ClN_3P$ (379,8)

| | | | | |
|---|---|---|---|---|
| Ber.: | C 66,41%; | H 5,04%; | N 11,06%; | Cl 9,34%; |
| Gef.: | C 64,56%; | H 5,04%; | N 11,38%; | Cl 9,68%. |

$^1$H-NMR ($CD_3COOD$): $\delta$ = 9,7 (s; 1H, 5-CH); 8,4 (s; 1H, 3-CH); 7,5 (m; 15H, $C_6H_5$) und 6,3 (d, $J_{PCH}$ = 7Hz; 2H, P-$CH_2$) ppm.

Beispiel 4: Herstellung von

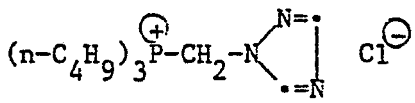

1H-1,2,4-Triazol-1-yl-methyl-tri-n-butylphosphoniumchlorid

Eine Lösung von 3,53 g (0,03 Mol) 1-Chlormethyl-1H-1,2,4-triazol und 6,1 g (0,03 Mol) Tributylphosphin in 30 ml Acetonitril wird 16 h unter Rückfluss erhitzt. Nach dem Abdampfen des Lösungsmittels erhält man 9,7 g (100%) 1H-1,2,4-Triazol-1-yl-methyl-tri-n-butylphosphoniumchlorid als wachsartige Masse.

$^1$H-NMR ($CD_3OC$); $\delta$ = 8,7 (s; 1H, 5-CH); 7,8 (s; 1H, 3-CH); 5,5 (d, $J_{PCH}$ = 7Hz; 2H, P-$CH_2$) und 0,5-2,7 (m; 27H, $C_4H_9$) ppm.

Beispiel 5: Herstellung von

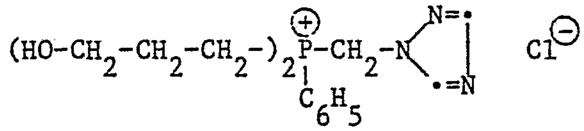

1H-1,2,4-Triazol-1-yl-methyl-di-(3-hydroxypropyl)-phenylphosphonium-chlorid

Eine Lösung von 3,53 g (0,03 Mol 1-Chlormethyl-1H-1,2,4-triazol und

6,88 g (0,03 Mol) Di-(3-hydroxypropyl)-phenyl-phosphin in 50 ml Acetonitril wird 16 h unter Rückfluss erhitzt. Nach dem Abdampfen des Lösungsmittels erhält man 10 g (98,5%) 1H-1,2,4-Triazol-1-yl-methyl-di-(3-hydroxypropyl)-phenylphosphoniumchlorid als hochviskoses Oel.

$^1$H-NMR ($CD_3OD$): $\delta$ = 8,2 (s; 1H, 5-CH); 7,7 (s; 1H, 3-CH); 7,4 (m; 5H, $C_6H_5$; 5,67 d, $J_{PCH}$ = 7Hz; 2H, P-$CH_2$-N); 4,3 (s; 2H, OH); 3,3 (m; 4H, O-$CH_2$); 2,6 (m; 4H, P-$CH_2$) und 1,6 (m; 4H, $CH_2$) ppm.

Beispiel 6: Herstellung eines Vorproduktes

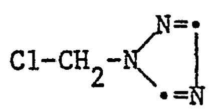

1-Chlormethyl-1H-1,2,4-triazol

a)Beim Erhitzen einer Mischung von 140 g (2,0 Mol) 1H-1,2,4-Triazol, 60 g (2,0 Mol) Paraformaldehyd und 1,5 ml Triäthylamin für 2h entsteht eine klare Schmelze, die beim Abkühlen erstarrt. Man erhält durch Umkristallisieren aus Aceton 195,3 g (98,5%) 1-Hydroxymethyl-1H-1,2,4-triazol, Fp. 65-67°.

$C_3H_5N_3O$ (99,1)

Ber.: C 36,3%; H 5,0%; N 42,4%;

Gef.: C 36,0%; H 5,0%; N 42,4%

$^1$H-NMR ($CDCl_3$): $\delta$= 8,47 (s; 1H, 5-CH); 8,0 (s; 1H, 3-CH); 7,35 (s; 1H, OH) und 5,67 (s; 2H, $CH_2O$) ppm.

b) Zu 600 ml Thionylchlorid lässt man innerhalb von 1,5 Stunden eine Schmelze von 195,3 g (1,975 Mol) 1-Hydroxymethyl-1H-1,2,4-triazol zutropfen. Es setzt eine starke Gasentwicklung ein und die Reaktionsmischung beginnt zu sieden. Nachdem die Schmelze vollständig zugesetzt worden ist, wird unter Kochen am Rückfluss noch für 2 Stunden gerührt. Nach dem Abkühlen wird das ausgefallene, gelbe 1-Chlormethyl-1H-1,2,4-triazol.hydrochlorid abfiltriert und zweimal mit je 300 ml Diäthyläther gewaschen. Ausbeute 272 g (90,2%), Smp. 120-127°.

$C_3H_4ClN_3 \cdot HCl$ (154)

| | | | |
|---|---|---|---|
| Ber.: | C 23,40%; | H 3,27%; | N 27,29%; |
| Gef.: | C 23,40%; | H 3,5%; | N 27,30%, |

$^1$H-NMR ($CD_3OD$): $\delta$ = 9,77 (s; 1H, 5-CH); 8,55 (s; 3-CH); 5,9 (s; 2H, $CH_2$-Cl) und 5,06 (s; 1H, HCl) ppm.

c) Zu 240,6 g (1,56 Mol) 1-Chlormethyl-1H-1,2,4-triazol-hydrochlorid in einem Gemisch von 600 ml Wasser und 400 ml Chloroform lässt man unter kräftigem Rühren 80 g Natriumhydroxid in 250 ml Wasser zutropfen. Nach Sättigen mit Natriumchlorid wird dreimal mit Chloroform extrahiert. Durch Trocknen, Eindampfen der Chloroformextrakte und fraktionierte Destillation des Rückstandes erhält man 156,2 g (85,1%) 1-Chlormethyl-1H-1,2,4-triazol, Sdp. 52-54°/0,27 mb.

$^1$H-NMR ($CDCl_3$): $\delta$ = 8,8 (s; 1H, 5-CH); 8,3 (s; 1H, 3-CH) und 6,3 (s; 2H, $CH_2Cl$) ppm.

Beispiel 7: (Zwischenprodukt)

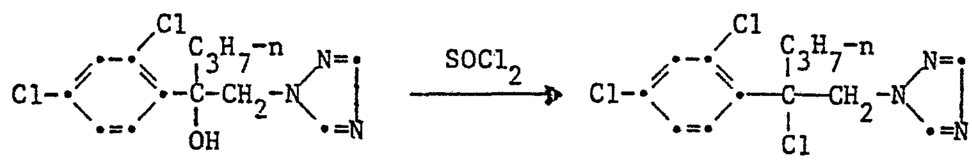

(4.1)

a) Herstellung von 1-(1H-1,2,4-Triazol-1'-yl)-2-chloro-2-2,4-dichlorphenyl)-pentan

6 g 1-(1H-1,2,4-Triazol-1'-yl)-2-(2,4-dichlorphenyl)-pentan-2-ol

werden in 50 ml Methylenchlorid gelöst und anschliessend tropfenweise mit 3,5 ml Thionylchlorid versetzt. Das Reaktionsgemisch wird 6 h unter Rückfluss erhitzt und eingedampft. Der Rückstand wird in Ethylacetat gelöst und nach Eiszugabe mit Sodalösung alkalisch eingestellt. Die organische Phase wird abgetrennt, über Natriumsulfat getrocknet und eingedampft. Der verbleibende harzige Rückstand wird durch Zugabe mit Petrolether zur Kristallisation gebracht, abfiltriert und aus Aceton-Petrolether umkristallisiert. Fp. 84-85°C.

b) Herstellung des reinen Isomeren des Endproduktes

(4.1) 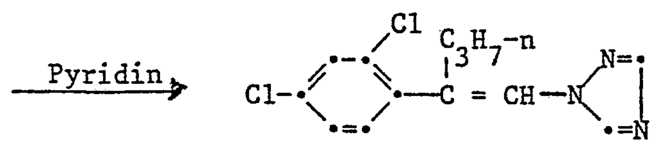


(1.1 b)

E-1-(1H-1,2,4-Triazol-1'-yl)-2-(2,4-dichlorphenyl)-penten-1

15 g des nach a) hergestellten rohen 1-(1H-1,2,4-Triazol-1'-yl)-2-chloro-2-(2,4-dichlorphenyl)-pentan werden in 50 ml Methylenchlorid und 30 ml Pyridin gelöst und 3 h unter Rückfluss und unter Rühren erhitzt. Das Methylenchlorid und überschüssiges Pyridin werden im Vakuum abdestilliert und der Rückstand mit eiskalter 4N-Natronlauge digeriert. Das Gemisch wird mit Diethylether extrahiert, die vereinigten Extrakte über Natriumsulfat getrocknet, filtriert und eingedampft. Der ölige Rückstand wird durch Zugabe von Petrolether zur Kristallisation gebracht. Man erhält 7,8 g E-1-(1H-1,2,4-Triazol-1'-yl)-2-(2,4-dichlorphenyl)-penten-1. Fp. 62-64°. Die Zuordnung des E-Isomeren erfolgt NMR-spektroskopisch.

Herstellungsbeispiele für Endprodukte:

Beispiel 8:

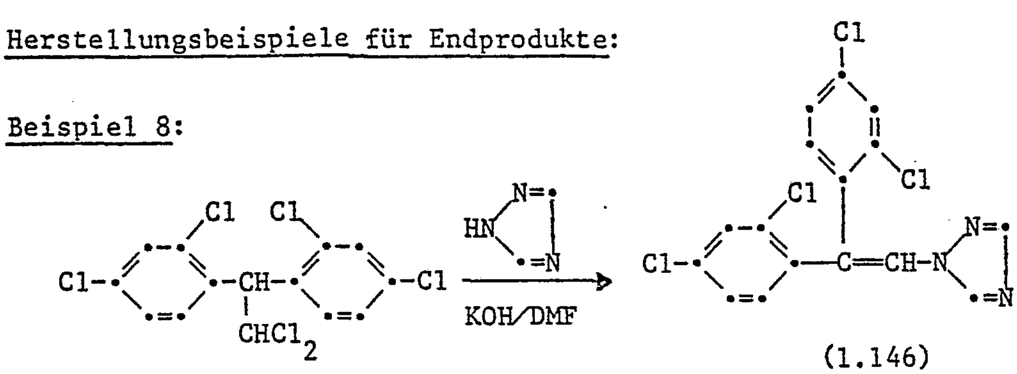


(1.146)

Herstellung von α-Bis(2,4-Dichlorphenyl)-β-(1H-1,2,4-triazol-1'-yl)-ethen

Eine Lösung bestehend aus 21,3 g 1-Bis(2,4-Dichlorphenyl)-2-dichlorethan, 13,8 g 1H-1,2,4-Triazol und 50 g pulverisiertes Kaliumhydroxid in 100 ml absolutem Dimethylformamid (DMF) wird 20 h auf 90° bis 100°C erwärmt, anschliessend auf Raumtemperatur abgekühlt, auf Eiswasser gegossen und mit Methylenchlorid extrahiert. Die vereinigten Extrakte werden mit Wasser gewaschen, über Natriumsulfat getrocknet und eingedampft. Der harzige Rückstand wird säulenchromatographisch (Kieselgel/Diethylether/n-Hexan; 1:1) gereinigt und aus Cyclohexan-Diethylether umkristallisiert, Fp. 103-106°C.

Beispiel 9: (Endprodukt)

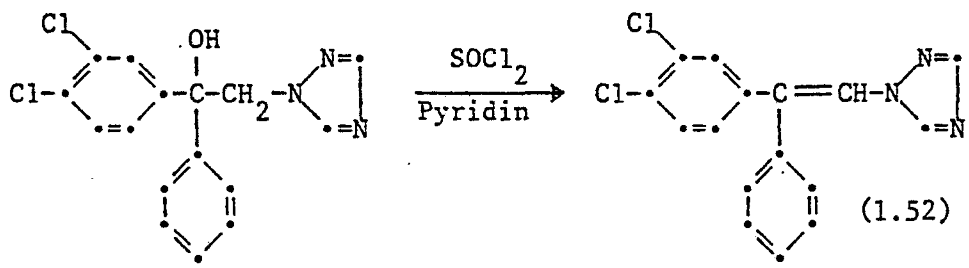


(1.52)

Herstellung von α-(3,4-Dichlorphenyl)-β-(1H-1,2,4-triazol-1'-yl)-styrol

13,4 g 1-[2-(3,4-Dichlorphenyl)-2-hydroxy-2-phenylethyl]-1H-1,2,4-triazol werden in 100 ml Pyridin gelöst. Zu dieser Lösung lässt man unter Eiskühlung bei + 10° bis +20°C langsam 29,1 ml Thionylchlorid zutropfen und rührt anschliessend das dunkle Reaktionsgemisch 16 h bei Raumtemperatur und 3 h bei +40°C. Nach Abkühlen auf Raumtemperatur wird das Gemisch auf Eiswasser gegossen, mit 2 N Salzsäure auf pH 2 angesäuert und 2 mal mit Ethylacetat extrahiert. Die vereinigten Extrakte werden 2 mal mit 1N Salzsäure, 2 mal mit halbgesättigter Kochsalzlösung und 1 mal mit gesättigter Natriumhydrogencarbonatlösung gewaschen, über Natriumsulfat getrocknet, filtriert und das Filtrat auf dem Wasserbad eingeengt. Das Rohprodukt wird säulenchromatographisch (Kieselgel/Dichlormethan) gereinigt und aus Ethylacetat/Petrolether umkristallisiert. Man erhält ein E/Z-Gemisch. Fp. 85-105°C.

Beispiel 10: Herstellung des Endproduktes

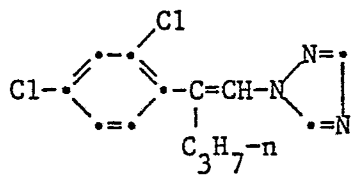

(1.1)

1-(1H-1,2,4-Triazol-1-yl)-2-(2,4-dichlorphenyl)-1-penten

a) Unter einer Stickstoffatmosphäre lässt man unter Feuchtigskeitsausschluss zur auf 5° bis 7° gekühlten Suspension von 1,6 g 55%igem Natriumhydrid in 15 ml Dimethoxymethan 7,8 g (0,036 Mol) Diethyl-(1H-1,2,4-triazol-1-yl)-methylphosphonat zutropfen. Nachdem nach 2 h Rühren bei Raumtemperatur die Wasserstoffentwicklung beendet ist, wird die Reaktionsmischung wieder auf 8° bis 9°C gekühlt und tropfenweise mit einer Lösung von 6,5 g (0,03 Mol) 2,4-Dichlorphenyl-n-propylketon in 15 ml Dimethoxyethan versetzt. Danach wird die Mischung für 18 h auf 50° erwärmt, anschliessend mit Eiswasser versetzt und dreimal mit Methylenchlorid extrahiert. Die Extrakte werden mit Wasser gewaschen, getrocknet und eingedampft. Durch fraktionierte Destillation erhält man 6,5 g (76,8%) 1-(1H-1,2,4-Triazol-1-yl)-2-(2,4-dichlorphenyl)-1-penten als E-Z-Isomerengemisch, Kp. 135-137°C/0,42 mb.

$C_{13}H_{13}Cl_2N_3$ (282,2)

| | | | |
|---|---|---|---|
| Ber.: | C 55,34%; | H 4,65%; | N 14,89%; |
| Gef.: | C 55,2%; | H 4,7%; | N 14,9%. |

b) Unter einer Stickstoffatmosphäre setzt man unter Feuchtigskeitsausschluss einer Suspension von 1,0 g 55%igem Natriumhydrid und einer Spatelspitze [15]-Krone-5 in 50 ml Dimethoxyethan in 2 Portionen insgesamt 7,6 g (0,02 Mol) kristallines 1H-1,2,4-Triazol-1-yl-methyl-triphenylphosphoniumchlorid zu. Die Reaktionsmischung nimmt eine orangerote Färbung an und die Temperatur steigt auf ca. 25° an. Nach Beendigung der Wasserstoffentwicklung lässt man

noch ca. 3 Stunden 4,3 g (0,02 Mol) 2,4-Dichlorphenyl-n-propylketon in 10 ml Dimethoxymethan zutropfen. Die Reaktionstemperatur steigt dabei auf ca.35° an. Nach 18 h Rühren wird die Reaktionsmischung mit Eiswasser versetzt und dreimal mit Diethylether extrahiert. Die Extrakte werden mit Wasser gewaschen, getrocknet und eingeengt. Das ausgefallene Triphenylphosphinoxid wird abgetrennt und die konzentrierte Lösung durch Filtration über eine kurze Kieselgelsäule gereinigt. Eindampfen des Filtrats ergibt 4,0 g (74,7%) 1-(1H-1,2,4-Triazol-1-yl)-2-(2,4-dichlorphenyl)-1-penten, das nach Dünnschichtchromatogramm und $^1$H-NMR-Spektrum identisch ist mit authentischem Material.

Beispiel 11: Herstellung von

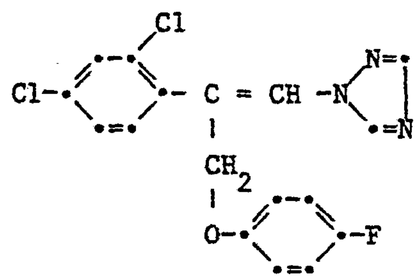

1-(1H-1,2,4-Triazol-1'-yl)-2-(2,4-dichlorphenyl)-3-(4-fluorphenoxy)-propen

Unter Schutzgasatmosphäre (Stickstoff) und unter Feuchtigkeitsausschluss werden 3,2 g einer 55 %igen Natriumhydrid-Dispersion in Mineralöl mit Petrolether digeriert. Der Petrolether wird abdekantiert und der ganze Vorgang noch zweimal wiederholt. Anschliessend werden 100 ml Dimethoxyethan und 0,1 g [18]-Krone-6 zugegeben und die Suspension auf 45° erwärmt. Nun lässt man unter Rühren eine Lösung von 17 g 1-[2,4-Dichlorphenyl]-2-[4-fluorphenoxy]-ethanon und 16,2 g Diethyl-(1H-1,2,4-triazol-1-yl)-methylphosphonat in 100 ml Dimethoxyethan zutropfen und rührt das Reaktionsgemisch 14 h bei 45°, kühlt auf 0° ab, giesst das Gemisch auf Eiswasser und rührt es 1,5 h bei 5 bis 10°. Daraufhin wird zweimal mit Essigsäureethylester extrahiert, die organische Phase mit Wasser gewaschen, über Natriumsulfat getrocknet,

filtriert und eingedampft. Der Rückstand wird säulenchromatographisch [Kieselgel/Diethylether] gereinigt und liefert 11,6 g (61 % der Theorie) 1-(1H-1,2,4-Triazol-1'-yl)-2-(2,4-dichlorphenyl)-3-(4-fluorphenoxy)propen als E/Z-Isomerengemisch.

Auf analoge Weise werden auch die nachfolgenden Verbindungen aus den Tabellen I bis IV hergestellt:

Tabelle I: Verbindung der Formel

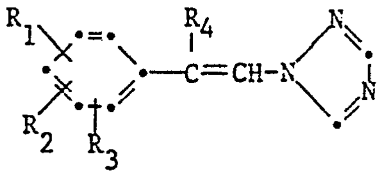

(Ia)

| No. | $R_1$ | $R_2$ | $R_3$ | $R_4$ | physik. Konst. |
|---|---|---|---|---|---|
| 1.1a | 2-Cl | 4-Cl | H | $C_3H_7$-n | Kp. 135-137°/ 0,42 mb (*) |
| 1.1b | 2-Cl | 4-Cl | H | $C_3H_7$-n | Fp. 64-66° (**) |
| 1.2 | 2-Cl | 4-Cl | H | $C_4H_9$-n | |
| 1.3 | 2-Cl | 4-Cl | H | $C_3H_7$-i | Oel |
| 1.4 | 2-Cl | 4-Cl | H | $C_2H_5$ | Oel |
| 1.5 | 2-Cl | 4-Cl | H | $CH_3$ | Oel |
| 1.6 | 2-Cl | 4-Cl | H | $C(CH_3)_3$ | |
| 1.7 | 2-Cl | 4-Cl | H | $C_6H_{13}$-n | |
| 1.8 | 2-Cl | 4-Cl | H | $C_{10}$-$H_{21}$-n | |
| 1.9 | 2-Cl | 4-Cl | H | 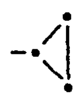 | Harz |
| 1.10 | 2-Cl | 4-Cl | H | 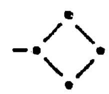 | |
| 1.11 | 2-Cl | 4-Cl | H | 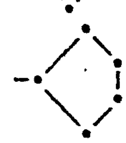 | |
| 1.12 | 2-Cl | 4-Cl | H | 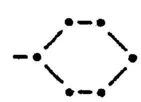 | |
| 1.13 | 3-Cl | 4-Cl | H | $-CH_3$ | halbfest |
| 1.14 | 2-Cl | 4-Cl | H | sek.-$C_4H_9$ | |
| 1.15 | 2-Cl | 4-Cl | H | $C_5H_{11}$-n | |
| 1.16 | 2-Cl | 4-Cl | H | Cycloheptyl | |
| 1.17 | H | 4-F | H | Cyclohexyl | |

(*) = E/Z-Gemisch; (**) = E-Form

Tabelle I (Fortsetzung)

| No. | $R_1$ | $R_2$ | $R_3$ | $R_4$ | physik. Konst. |
|---|---|---|---|---|---|
| 1.18 | 2-Cl | 4-Cl | H | 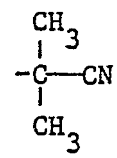 | |
| 1.19 | 2-Cl | 4-Cl | H | 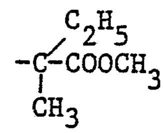 | |
| 1.20 | 2-Cl | 4-Cl | H | Cyclohexyl | |
| 1.21 | 2-Cl | 4-F | H | Cycloheptyl | |
| 1.22 | 2-Cl | 4-Cl | H | $CH_2$-Cyclohexyl | |
| 1.23 | 4-Cl | H | H | $CH_3$ | |
| 1.24 | 4-Cl | H | H | $C_2H_5$ | |
| 1.25 | 4-Cl | H | H | $C_3H_7$-n | Oel |
| 1.26 | 4-Cl | H | H | $C_4H_9$-n | |
| 1.27 | 4-Cl | H | H | 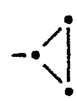 | Fp. 60-62° |
| 1.28 | 4-Cl | H | H | 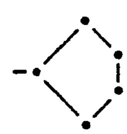 | |
| 1.29 | 4-Cl | H | H | 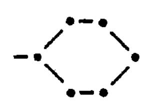 | Fp. 100-103° |
| 1.30 | 4-Cl | H | H | sek.-$C_4H_9$ | |
| 1.31 | 4-Cl | H | H | tert.-$C_4H_9$ | zähes Harz |
| 1.32 | 4-Cl | H | H | $C_5H_{11}$-n | |
| 1.33 | 4-Cl | H | H | 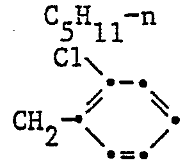 | |

Tabelle I (Fortsetzung)

| No. | $R_1$ | $R_2$ | $R_3$ | $R_4$ | physik. Konst. |
|---|---|---|---|---|---|
| 1.34 | 4-Cl | H | H | 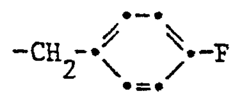 | |
| 1.35 | 4-Cl | H | H | 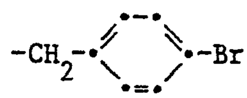 | |
| 1.36 | 2-Cl | 4-Cl | H | 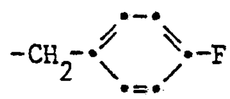 | |
| 1.37 | 2-Cl | 4-Cl | H | $-CH=CH-CH_3$ | |
| 1.38 | 2-Cl | 4-Cl | H | 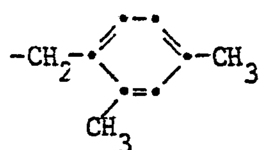 | |
| 1.39 | 2-Cl | 4-Cl | H | 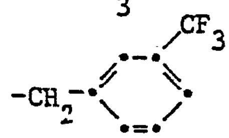 | |
| 1.40 | 2-Cl | 4-Cl | 6-Cl | 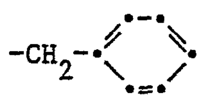 | |
| 1.41 | 2-Cl | 4-Cl | H | 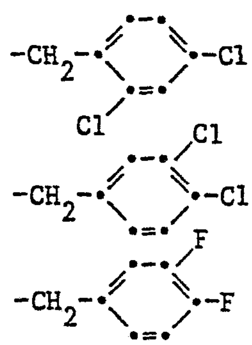 | |
| 1.42 | 2-Cl | 4-Cl | H | | |
| 1.43 | 2-Cl | 4-Cl | H | | |
| 1.44 | 2-Cl | 4-Cl | H | sek.-$C_4H_9$ | |
| 1.45 | 2-Cl | 4-Cl | H | tert.-$C_4H_9$ | |
| 1.46 | 2-Cl | 4-Cl | H | $C_5H_{11}$-n | |
| 1.47 | 2-Cl | 4-Cl | H | 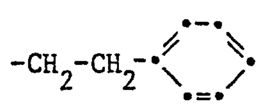 | |

Tabelle I (Fortsetzung)

| No. | $R_1$ | $R_2$ | $R_3$ | $R_4$ | physik. Konst. |
|---|---|---|---|---|---|
| 1.48 | 2-Cl | 4-Cl | H | sek.-$C_4H_9$ | |
| 1.49 | 2-Cl | 4-Cl | H | tert.-$C_4H_9$ | |
| 1.50 | 2-Cl | 4-Cl | H | $C_5H_{11}$-n | |
| 1.51 | 2-Cl | 4-Cl | H | 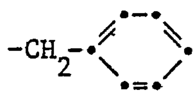 | |
| 1.52 | 2-Cl | 4-Cl | H | 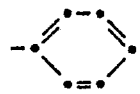 | Oel |
| 1.53 | 2-Cl | 4-Cl | H | 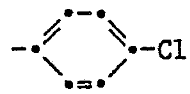 | |
| 1.54 | 2-Cl | 4-Cl | H | 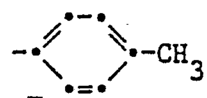 | |
| 1.55 | 2-Cl | 4-Cl | H | 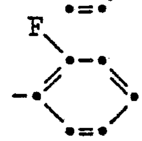 | |
| 1.56 | 2-Cl | 4-Br | H | $C_3H_7$-n | |
| 1.57 | 2-Cl | 4-Br | H | $C_2H_5$ | |
| 1.58 | 2-Cl | 4-Br | H | $CH_3$ | |
| 1.59 | 2-Cl | 4-Br | H | $C_4H_9$-n | |
| 1.60 | 2-Cl | 4-Br | H | 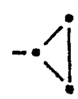 | |
| 1.61 | 2-Cl | 4-Br | H | 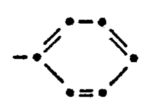 | |
| 1.62 | 2-Cl | 4-Br | H | sek.-$C_4H_9$ | |
| 1.63 | 2-Cl | 4-Br | H | tert.-$C_4H_9$ | |
| 1.64 | 2-Cl | 4-Br | H | 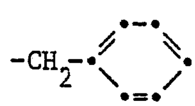 | |

Tabelle I (Fortsetzung)

| No. | $R_1$ | $R_2$ | $R_3$ | $R_4$ | physik. Konst. |
|---|---|---|---|---|---|
| 1.65 | 2-Cl | 4-Br | H | 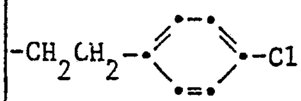 | |
| 1.66 | 2-Cl | 4-Br | H | 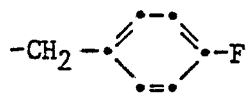 | |
| 1.67 | 2-Cl | 4-F | H | $C_3H_7$ | |
| 1.68 | 2-Cl | 4-F | H | $C_2H_5$ | |
| 1.69 | 2-Cl | 4-F | H | 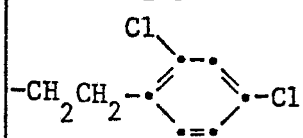 | |
| 1.70 | 2-Cl | 4-F | H | 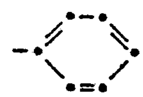 | |
| 1.71 | H | H | H | 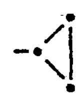 | Oel |
| 1.72 | 4-Cl | H | H | 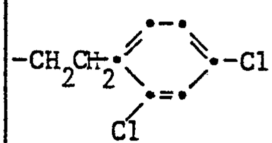 | |
| 1.73 | 4-Cl | H | H | 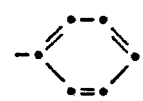 | Fp. 98-118° |
| 1.74 | 4-Cl | H | H | 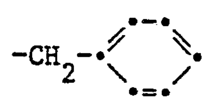 | |
| 1.75 | 4-$OCH_3$ | H | H | $-C_2H_5$ | Oel |
| 1.76 | 4-$OCH_3$ | H | H | 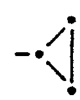 | Fp. 82-83° |
| 1.77 | 4-$OCH_3$ | H | H | 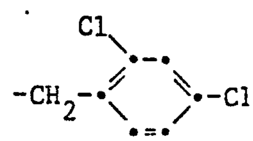 | |
| 1.78 | 4-Cl | H | H | 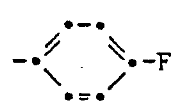 | Fp. 100-102° |

Tabelle I (Fortsetzung)

| No. | $R_1$ | $R_2$ | $R_3$ | $R_4$ | physik. Konst. |
|---|---|---|---|---|---|
| 1.79 | 2-$OCH_3$ | 4-$OCH_3$ | H | 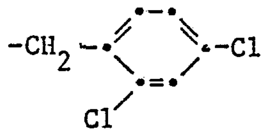 | |
| 1.80 | 3-Cl | 4-Cl | H | 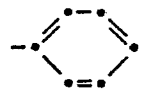 | Fp. 85-105° |
| 1.81 | 3-Cl | 4-Cl | H | $-C_3H_7$-n | |
| 1.82 | 3-Cl | 4-Cl | H | 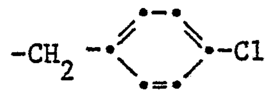 | |
| 1.83 | 3-Cl | 4-Cl | H | 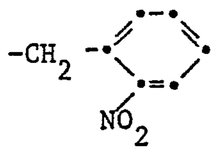 | |
| 1.84 | 2-$NO_2$ | H | H | $-CH_3$ | |
| 1.85 | 3-$NO_2$ | H | H | $-CH_3$ | |
| 1.86 | 4-$NO_2$ | H | H | $-C_3H_7$-n | |
| 1.87 | 3-$NO_2$ | 4-Cl | H | $-CH_3$ | |
| 1.88 | 2-F | H | H | $-C_2H_5$ | |
| 1.89 | 2-F | H | H | $-C_3H_7$-n | |
| 1.90 | 2-F | H | H | $-C_4H_9$ | |
| 1.91 | 2-F | H | H | 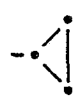 | |
| 1.92 | 2-F | H | H | 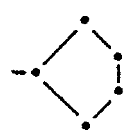 | |
| 1.93 | 2-F | H | H | 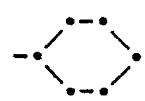 | |

Tabelle I (Fortsetzung)

| No. | $R_1$ | $R_2$ | $R_3$ | $R_4$ | physik. Konst. |
|---|---|---|---|---|---|
| 1.94 | 2-F | H | H | $-CH_2$ 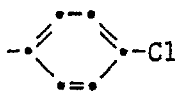 | |
| 1.95 | 2-F | H | H | $-CH_2-C_2H_5$ | |
| 1.96 | 2-F | H | H | $-CH_2$ 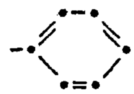 | |
| 1.97 | 2-F | H | H | 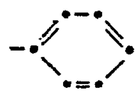 | |
| 1.98 | 4-F | H | H | $-CH_3$ | Oel |
| 1.99 | 4-F | H | H | $-C_3H_7$-n | |
| 1.100 | 4-F | H | H | 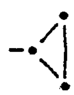 | |
| 1.101 | 4-F | H | H | 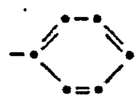 | Fp. 98-101° |
| 1.102 | 4-F | H | H | 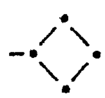 | |
| 1.103 | 2-$C_6H_5$ | H | H | $CH_3$ | |
| 1.104 | 2-$C_6H_5$ | H | H | $C_2H_5$ | |
| 1.105 | 2-$C_6H_5$ | 4-Cl | H | $C_3H_7$-i | |
| 1.106 | 2-$C_6H_5$ | 4-Cl | H | $C_3H_7$-i | |
| 1.107 | 2-Cl | 4-Cl | H | 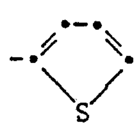 | |

Tabelle I (Fortsetzung)

| No. | $R_1$ | $R_2$ | $R_3$ | $R_4$ | physik. Konst. |
|---|---|---|---|---|---|
| 1.108 | 2-Cl | 4-Cl | 6-Cl | $-C_2H_5$ | |
| 1.109 | 2-Cl | 4-Cl | 6-Cl | $-C_3H_7$ | |
| 1.110 | 2-Cl | 4-Cl | 6-Cl | 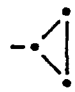 | |
| 1.111 | 2-Cl | H | H | 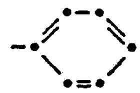 | Oel |
| 1.112 | 2-Cl | 4-Cl | 6-Cl | $-CH_2-$ 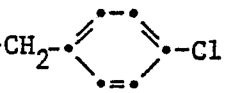 | |
| 1.113 | 2-Cl | 4-Cl | H | 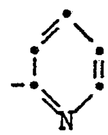 | |
| 1.114 | 2-Cl | 4-Cl | H | 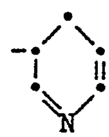 | |
| 1.115 | 4-Cl | | | 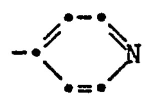 | |
| 1.116 | 4- 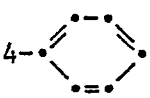 | H | H | $-CH_3$ | Fp. 100-105° |
| 1.117 | 4- 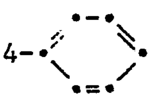 | H | H | $-C_2H_5$ | |
| 1.118 | 4- 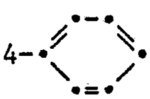 | H | H | $-C_3H_7$-n | |
| 1.119 | 4- 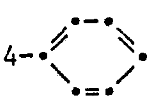 | H | H | 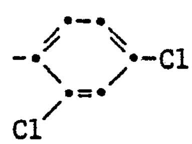 | |
| 1.120 | 4- 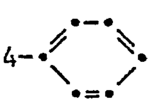 | H | H | $-CH_2-O-C_2H_5$ | |
| 1.121 | 4- 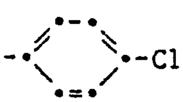 | H | H | $-CH_2-$ 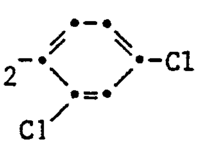 | |

0060223



Tabelle I (Fortsetzung)

| No. | $R_1$ | $R_2$ | $R_3$ | $R_4$ | physik. Konst. |
|---|---|---|---|---|---|
| 1.122 | 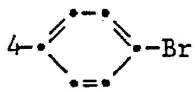 | H | H | 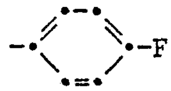 | |
| 1.123 | 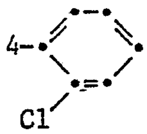 | H | H | $-C_3H_7$ | |
| 1.124 | 2-Cl | 4-Br | H | 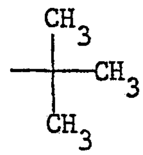 | |
| 1.125 | 4-Cl | 4-Br | H | 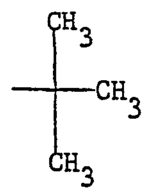 | |
| 1.126 | 2-Cl | 4-Cl | H | 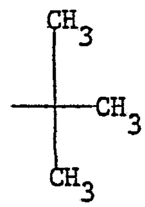 | |
| 1.127 | 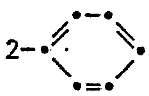 | H | H | $-C_3H_7$ | |
| 1.128 | 4-F | H | H | 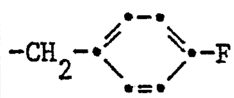 | |
| 1.129 | 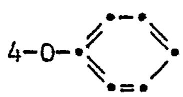 | H | H | $-C_2H_5$ | |
| 1.130 | 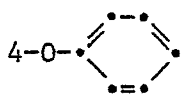 | H | H | $-C_3H_7$-n | |
| 1.131 | 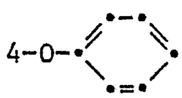 | H | H | 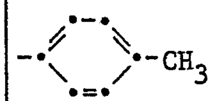 | halbfest |
| 1.132 | 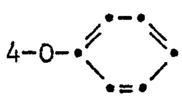 | H | H | 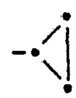 | |
| 1.133 | H | H | H | $-C_3H_7$ | |

Tabelle I (Fortsetzung)

| No. | $R_1$ | $R_2$ | $R_3$ | $R_4$ | physik. Konst. |
|---|---|---|---|---|---|
| 1.134 | H | H | H | $-CH_2CH=CH_2$ | |
| 1.135 | H | H | H | $-CH_2C\equiv CH$ | |
| 1.136 | 2-Cl | 4-Cl | H | $-CH_2CH=CH_2$ | |
| 1.137 | 2-Cl | 4-Cl | H | $-CH_2CH_2-$ 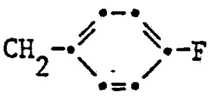 | |
| 1.138 | 2-Cl | 4-Cl | H | 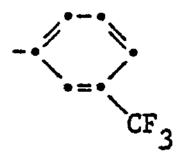 | |
| 1.139 | 2-Cl | 4-Cl | H | 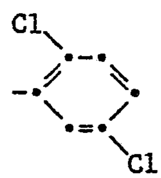 | |
| 1.140 | 2-F | 4-F | H | $CH_3$ | |
| 1.141 | 4-$NO_2$ | H | H | 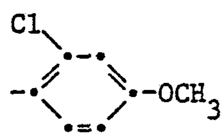 | |
| 1.142 | 2-$OCH_3$ | 4-$OCH_3$ | H | 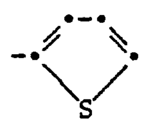 | |
| 1.143 | 4-Br | H | H | 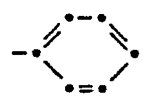 | Fp. 107-9° |
| 1.144 | H | H | H | 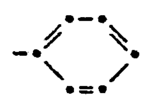 | Fp. 58-9° |
| 1.145 | 4-$CH_3$ | H | H | 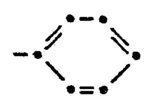 | Fp. 81-94° |
| 1.146 | 2-Cl | 4-Cl | H | 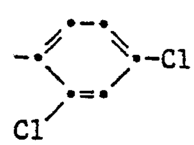 | Fp. 103-106° |
| 1.147 | 4-$C_6H_5$ | H | H | H | |

Tabelle I: (Fortsetzung)

| No. | $R_1$ | $R_2$ | $R_3$ | $R_4$ | physik. Konst. |
|---|---|---|---|---|---|
| 1.148 | 2-Cl | 4-Cl | H | $-CH_2OCH_3$ | |
| 1.149 | 2-Cl | 4-Cl | H | $-CH_2OC_2H_5$ | |
| 1.150 | 2-Cl | 4-Cl | H | $-CH_2O-C_3H_7-n$ | |
| 1.151 | 2-Cl | 4-Cl | H | $-CH_2O-C_4H_9-n$ | |
| 1.152 | 2-Cl | 4-Cl | H | $-CH_2O-C(CH_3)_2$ | |
| 1.153 | 2-Cl | 4-Br | H | $-C(CH_3)_2-CN$ | |
| 1.154 | 2-Cl | 4-Cl | H | $-CH_2O-C_6H_4F(4)$ | Oel |
| 1.155 | 2-Cl | 4-Cl | H | $-CH_2O-C_6H_4(C_4H_9-t)(4)$ | Oel |
| 1.156 | 2-Cl | 4-Cl | H | $-CH_2O-C_6H_4Cl(4)$ | Oel |
| 1.157 | 2-Cl | 4-Cl | H | $-CH_2O-C_6H_4Cl(2)$ | |
| 1.158 | 4-Cl | H | H | $-CH_2OCH_3$ | |
| 1.159 | 4-Cl | H | H | $-CH_2O-C_2H_5$ | |
| 1.160 | 4-Cl | H | H | $-CH_2O-C_3H_7-n$ | |
| 1.161 | 4-Cl | H | H | $-CH_2O-C_6H_4Cl(2)$ | Oel |
| 1.162 | 4-Cl | H | H | $-CH_2O-C_6H_4F(4)$ | |
| 1.163 | 4-Cl | H | H | $-CH_2O-C_6H_4Br(4)$ | |
| 1.164 | 2-Cl | 4-Cl | H | $-CH_2O-C_6H_4NO_2(4)$ | |
| 1.165 | 2-Cl | 4-Cl | H | $-CH_2O-C_6H_3(CH_3)_2(2,4)$ | |
| 1.166 | 2-Cl | 4-Cl | H | $-CH_2O-C_6H_3CF_3(3)$ | |
| 1.167 | 2-Cl | 4-Cl | H | $-CH_2CH_2OC_2H_5$ | |
| 1.168 | 2-Cl | 4-Cl | H | $-CH_2CH_2OCH_3$ | |
| 1.169 | 2-Cl | 4-Cl | H | $-CH_2CH_2SCH_3$ | |
| 1.170 | 2-Cl | 4-Cl | H | $-CH_2CH_2O-C_6H_5$ | |
| 1.171 | 2-Cl | 4-Cl | H | $-CH_2SCH_3$ | |
| 1.172 | 2-Cl | 4-Cl | H | $-CH_2SC_2H_5$ | |
| 1.173 | 2-Cl | 4-Cl | H | $-CH_2CH_2SCH_3$ | |

Tabelle I: (Fortsetzung)

| No. | $R_1$ | $R_2$ | $R_3$ | $R_4$ | physik. Konst. |
|---|---|---|---|---|---|
| 1.174 | 2-Cl | 4-Cl | H | $-CH_2O-C_6H_5$ | Oel |
| 1.175 | 2-Cl | 4-Br | H | $-CH_2OCH_3$ | |
| 1.176 | 2-Cl | 4-Br | H | $-CH_2OC_2H_5$ | |
| 1.177 | 2-Cl | 4-Br | H | $-CH_2O-C_6H_5$ | |
| 1.178 | 2-Cl | 4-Br | H | $-CH_2O-C_6H_4Cl(4)$ | |
| 1.179 | 2-Cl | 4-Br | H | $-CH_2O-C_6H_4F(4)$ | |
| 1.180 | 2-Cl | 4-F | H | $-CH_2O-C_6H_5Cl_2(2,4)$ | |
| 1.181 | $4-OCH_3$ | H | H | $-CH_2O-C_6H_3Cl_2(2,4)$ | Fp. 129÷130° |
| 1.182 | $4-OCH_3$ | H | H | $-CH_2O-C_6H_3Cl(4)$ | |
| 1.183 | 3-Cl | 4-Cl | H | $-CH_2O-C_6H_4Cl(4)$ | |
| 1.184 | 3-Cl | 4-Cl | H | $-CH_2O-C_6H_4NO_2(2)$ | |
| 1.185 | 2-F | H | H | $-CH_2O-C_6H_4Cl(4)$ | |
| 1.186 | 2-F | H | H | $-CH_2OC_2H_5$ | |
| 1.187 | 2-F | H | H | $-CH_2O-C_6H_5$ | |
| 1.188 | 4-F | H | H | $-CH_2O-C_6H_3Cl_2(2,4)$ | |
| 1.189 | 4-F | H | H | $-CH_2CH_2OCH_3$ | |
| 1.190 | 4-F | H | H | $-CH_2OCH_3$ | |
| 1.191 | 2-Cl | 4-Cl | H | $-C(CH_3)_2-COOCH_3$ | |
| 1.192 | 2-Cl | 4-Cl | H | $-C(C_2H_5)_2-CN$ | |
| 1.193 | 2-Cl | 4-Cl | 6-Cl | $-CH_2O-C_6H_4Cl(4)$ | |
| 1.194 | $4-C_6H_5$ | 4-Cl | 6-Cl | $-CH_2OC_4H_9-n$ | |
| 1.195 | 2-Cl | 4-Cl | H | $-CH_2CH_2O-C_6H_4F(4)$ | |
| 1.196 | 2-Cl | 4-Cl | H | $-CH_2O-C_6H_4CF_3(3)$ | |
| 1.197 | 2-Cl | 4-Cl | H | $-CH_2O-CH_2C_6H_5$ | |
| 1.198 | 4-F | H | H | $-CH_2O$-Cyclohexyl | |
| 1.199 | 2-Cl | 4-Cl | H | $-CH(C_3H_7-n)O-C_6H_4Cl(4)$ | |
| 1.200 | 2-Cl | 4-Cl | H | $-CH(C_2H_5)O-C_6H_5$ | |
| 1.201 | 4-Cl | H | H | $-CH_2O$-Cyclobutyl | |
| 1.202 | 4-Cl | H | H | $-CH_2O$-Cyclohexyl | |
| 1.203 | 2-Cl | 4-Cl | H | $-CH_2O$-Cyclohexyl | |

Tabelle II: Verbindungen der Formel

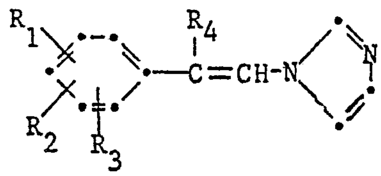

(Ib)

| No. | $R_1$ | $R_2$ | $R_3$ | $R_4$ | physik. Konst. |
|---|---|---|---|---|---|
| 2.1 | 4-Cl | H | H | 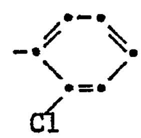 | Fp. 72-5° |
| 2.2 | H | H | H | 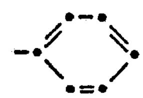 | Fp. 215-7° |
| 2.3 | 4-Cl | H | H | 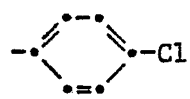 | Fp. 72-4° |
| 2.4 | $4-CH_3$ | H | H | 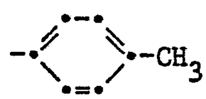 | Fp. 90-2° |
| 2.5 | 2-Cl | 4-Cl | H | $-CH_3$ | |
| 2.6 | 2-Cl | 4-Cl | H | $-C_2H_5$ | |
| 2.7 | 2-Cl | 4-Cl | H | $-C_3H_7-n$ | |
| 2.8 | 2-Cl | 4-Cl | H | 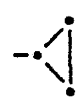 | Harz |
| 2.9 | 2-Cl | 4-Cl | H | 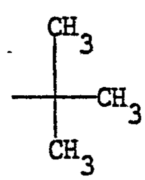 | |
| 2.10 | 2-Cl | 4-Cl | H | $-CH_2OCH_3$ | |
| 2.11 | 2-Cl | 4-Cl | H | 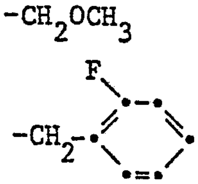 | |
| 2.12 | 2-Cl | 4-Cl | H | $-CH_2CH=CH_2$ | |

Tabelle II (Fortsetzung)

| No. | $R_1$ | $R_2$ | $R_3$ | $R_4$ | physik. Konst. |
|---|---|---|---|---|---|
| 2.13 | 2-Cl | 4-Cl | H | $-CH_2CH_2-$ 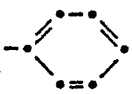 | |
| 2.14 | 4-Cl | H | H | 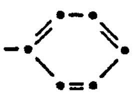 | |
| 2.15 | 4-Cl | H | H | $C_4H_9$-n | |
| 2.16 | 4-Cl | H | H | $C_5H_{11}$-n | |
| 2.17 | 4- 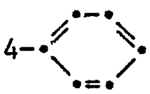 | H | H | $-C_2H_5$ | |
| 2.18 | 4- 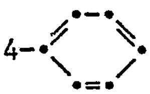 -Cl | H | H | $-C_3H_7$-n | |
| 2.19 | 4-O- 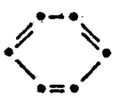 | H | H | $-C_3H_7$-i | |
| 2.20 | 4-O- 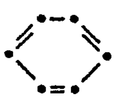 | H | H | $-CH_2-$ 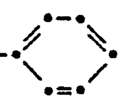 $-OCH_3$ | |
| 2.21 | 2-Cl | 4-Cl | H | Cyclohexyl | |
| 2.22 | H | 4-Cl | H | Cyclohexyl | |
| 2.23 | H | 4-F | H | Cyclohexyl | |
| 2.24 | H | 4-F | H | Cyclobutyl | |
| 2.25 | 2-Cl | 4-F | H | Cycloheptyl | |
| 2.26 | 2-Cl | 4-Cl | 6-Cl | Cyclohexyl | |
| 2.27 | $2-CH_3$ | 4-Cl | H | $C_3H_7$-n | |
| 2.28 | $2-CH_3$ | H | $6-CH_3$ | $C_3H_7$-i | |

Tabelle II: (Fortsetzung)

| No. | $R_1$ | $R_2$ | $R_3$ | $R_4$ | physik. Konst. |
|---|---|---|---|---|---|
| 2.29 | 2-Cl | 4-Cl | H | $-CH_2OCH_3$ | |
| 2.30 | 2-Cl | 4-Cl | H | $-CH_2OC_2H_5$ | |
| 2.31 | 2-Cl | 4-Cl | H | $-CH_2O-C_3H_7-n$ | |
| 2.32 | 2-Cl | 4-Cl | 6-Cl | $-CH_2O-C_4H_9-n$ | |
| 2.33 | 2-Cl | 4-Cl | H | $-CH_2O-C(CH_3)_2$ | |
| 2.34 | 2-Cl | 4-Cl | H | $-C(CH_3)_2-CN$ | |
| 2.35 | 2-Cl | 4-Cl | H | $-CH_2O-C_6H_4F(4)$ | |
| 2.36 | 2-Cl | 4-Cl | H | $-CH_2O-C_6H_4(C_4H_9-t)(4)$ | |
| 2.37 | 2-Cl | 4-Cl | H | $-CH_2O-C_6H_4Cl(4)$ | |
| 2.38 | 2-Cl | 4-Cl | H | $-CH_2O-C_6H_4Cl(2)$ | |
| 2.39 | 4-Cl | H | H | $-CH_2O-CH_3$ | |
| 2.40 | 4-Cl | H | H | $-CH_2O-C_2H_5$ | |
| 2.41 | 4-Cl | H | H | $-CH_2O$-Cyclohexyl | |
| 2.42 | 4-Cl | H | H | $-CH_2O-CH_2C_6H_5$ | |
| 2.43 | 4-Cl | H | H | $-CH_2O-C_6H_4F(4)$ | |
| 2.44 | 4-Cl | H | H | $-CH_2O-C_6H_4Br(4)$ | |
| 2.45 | 2-Cl | 4-Cl | H | $-CH_2O-C_6H_4NO_2(4)$ | |
| 2.46 | 2-Cl | 4-Cl | H | $-CH_2O-C_6H_3(CH_3)_2(2,4)$ | |
| 2.47 | 2-Cl | 4-Cl | H | $-CH_2O-C_6H_3CF_3(3)$ | |
| 2.48 | 2-Cl | 4-Cl | H | $-CH(C_2H_5)O-C_6H_5$ | |
| 2.49 | 2-Cl | 4-Cl | H | $-CH_2CH_2OCH_3$ | |
| 2.50 | 2-Cl | 4-Cl | H | $-CH_2CH_2SCH_3$ | |
| 2.51 | 2-Cl | 4-Cl | H | $-CH_2CH_2O-C_6H_5$ | |

Tabelle III: Verbindungen der Formel

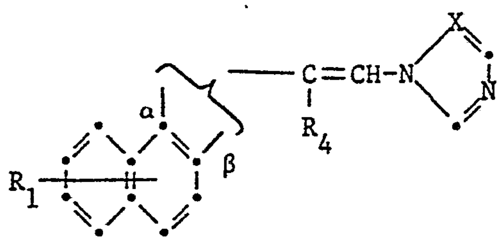

(Ic)

| No. | $R_1$ | Ver-knüpfung | $R_4$ | X | physik. Konst. |
|---|---|---|---|---|---|
| 3.1 | H | α | $-C_2H_5$ | N | |
| 3.2 | H | α | $-C_3H_7$-n | N | semikristallin |
| 3.3 | H | α | 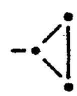 | N | |
| 3.4 | H | α | $-C_4H_9$-n | CH | |
| 3.5 | H | α | $-CH_3$ | CH | |
| 3.6 | 2-$OCH_3$ | α | $-C_2H_5$ | N | |
| 3.7 | 4-F | α | $-C_3H_7$-n | N | |
| 3.8 | 4-Cl | α | $-C_4H_9$-n | CH | |
| 3.9 | 4-Cl | α | $C_3H_7$-n | CH | |
| 3.10 | 4-$OCH_3$ | α | $-CH_3$ | N | |
| 3.11 | H | β | $-CH_3$ | N | |
| 3.12 | H | β | 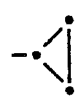 | CH | |
| 3.13 | 5,8-Di-Cl | α | 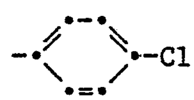 | N | |
| 3.14 | H | α | H | N | |
| 3.15 | H | β | H | N | |

Tabelle III: (Fortsetzung)

| No. | $R_1$ | Verknüpfung | $R_4$ | X | physik. Konst. |
|---|---|---|---|---|---|
| 3.16 | H | α | $-CH_2OCH_3$ | N | |
| 3.17 | H | α | $-CH_2OC_2H_5$ | N | |
| 3.18 | H | α | $-CH_2O-C(CH_3)_2$ | N | |
| 3.19 | H | α | $-CH_2OCH_3$ | CH | |
| 3.20 | H | α | $-CH_2O-C_6H_5$ | CH | |
| 3.21 | $2-OCH_3$ | α | $-CH_2O-C_6H_4Cl(4)$ | N | |
| 3.22 | 4-F | α | $-CH_2O-C_6H_4F(4)$ | N | |
| 3.23 | 4-Cl | α | $-CH_2O-C_6H_4CH_3(4)$ | CH | |
| 3.24 | 4-Cl | α | $-CH_2CH_2OCH_3$ | CH | |
| 3.25 | $4-OCH_3$ | α | $-CH_2CH_2SCH_3$ | N | |
| 3.26 | H | β | $-CH_2OCH_3$ | N | |
| 3.27 | H | β | $-CH_2OCH_3$ | CH | |
| 3.28 | 5,8-Di-Cl | α | $-CH_2OCH_3$ | N | |
| 3.29 | H | α | $-CH_2O-C_6H_3Cl_2(2,4)$ | N | |
| 3.30 | H | β | $-CH_2O-C_6H_3Cl_2(2 4)$ | N | |

Tabelle IV: Verbindungen der Formel

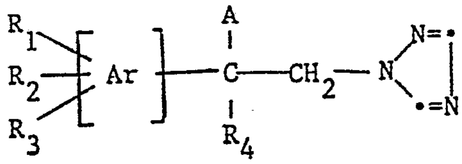

| No. | 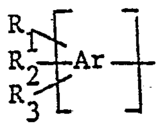 | $R_4$ | A | phys. Konst. |
|---|---|---|---|---|
| 4.1 | 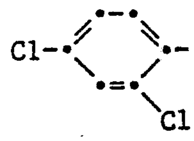 | $-C_3H_7(n)$ | -Cl | Fp. 84-85° |
| 4.2 | 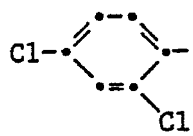 | $-C_3H_7(n)$ | -Br | zähes Harz |
| 4.3 | 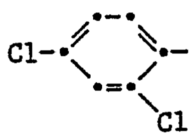 | $-C_3H_7(n)$ | $-OSO_2CH_3$ | |
| 4.4 | 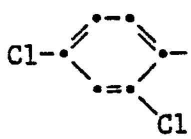 | $-C_3H_7(n)$ | $-OCOCH_3$ | Oel |
| 4.5 | 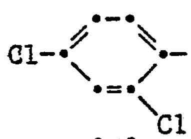 | $-C_3H_7(n)$ | 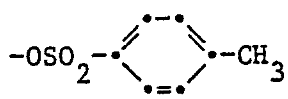 | |
| 4.6 | 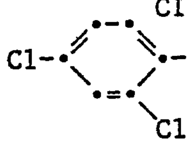 | $-CH_3$ | -Cl | |
| 4.7 | 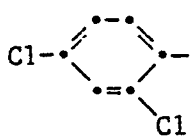 | $-C_4H_9(n)$ | -Cl | |
| 4.8 | 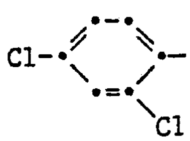 | $-C_4H_9(t)$ | -Cl | |
| 4.9 | 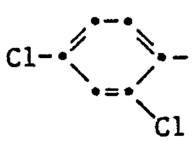 | 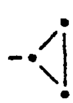 | -Cl | Harz |

Tabelle IV (Fortsetzung)

| No. | 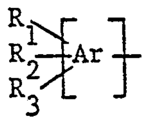 | $R_4$ | A | phys. Konst. |
|---|---|---|---|---|
| 4.10 | 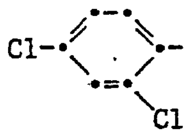 | 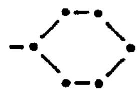 | -Cl | |
| 4.11 | 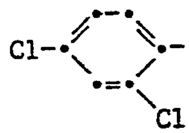 | 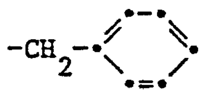 | -Cl | |
| 4.12 | 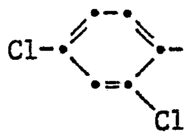 | 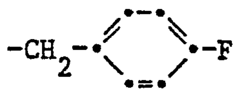 | -Cl | |
| 4.13 | 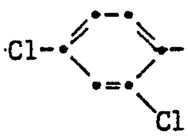 | $C_2H_5$ | -Cl | zähes Harz |
| 4.14 | 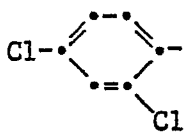 | $C_4H_9$-s | -J | |
| 4.15 | 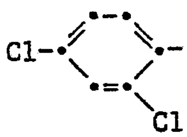 | 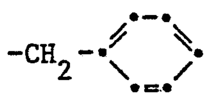 | -Cl | |
| 4.16 | 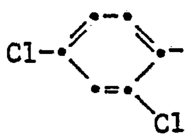 | 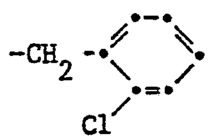 | -Cl | |
| 4.17 | 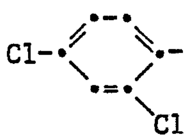 | 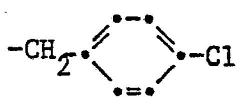 | -Cl | Oel |
| 4.18 | 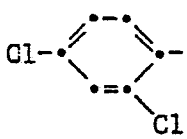 | 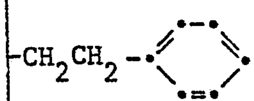 | $-OCOCH_3$ | |
| 4.19 | 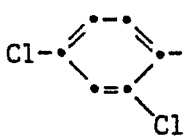 | 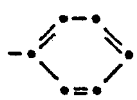 | -Cl | |

Tabelle IV (Fortsetzung)

| No. | 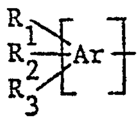 | $R_4$ | A | phys. Konst. |
|---|---|---|---|---|
| 4.20 | 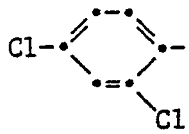 | $-C_3H_7-n$ | -J | |
| 4.21 | 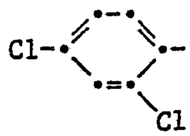 | 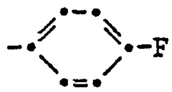 | -Cl | |
| 4.22 | 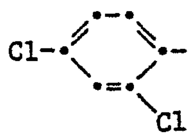 | 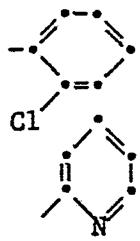 | -Cl | |
| 4.23 | 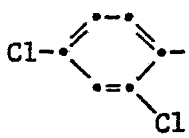 | | $-OCOC_2H_5$ | |
| 4.24 | 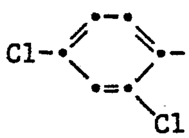 | 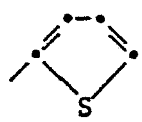 | -Cl | |
| 4.25 | 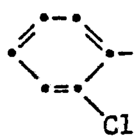 | $C_4H_9(t)$ | -Cl | |
| 4.26 | 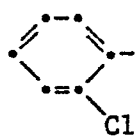 | $C_4H_9(t)$ | -Br | |
| 4.27 | 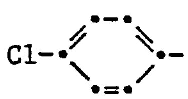 | $C_4H_9(n)$ | -Cl | |
| 4.28 | 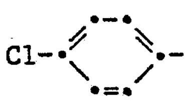 | $C_4H_9(n)$ | 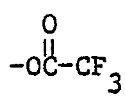 | |
| 4.29 | 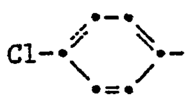 | 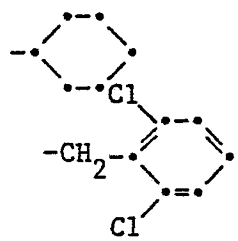 | -Cl | |
| 4.30 | 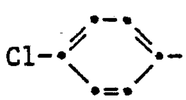 | | -Cl | |

Tabelle IV (Fortsetzung)

| No. | 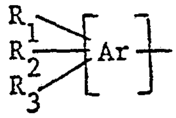 | $R_4$ | A | phys. Konst. |
|---|---|---|---|---|
| 4.31 | 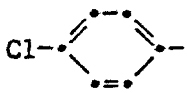 | 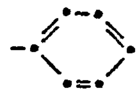 | -Cl | |
| 4.32 | 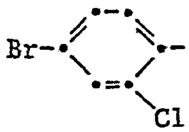 | $-C_3H_7(n)$ | -Cl | |
| 4.33 | 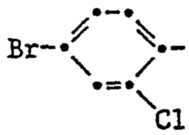 | $-C_4H_9(t)$ | -Cl | |
| 4.34 | 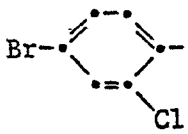 | 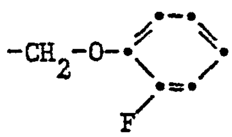 | $-OCOCH_3$ | |
| 4.35 | 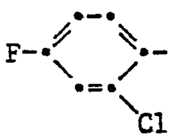 | 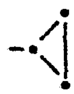 | -Cl | |
| 4.36 | 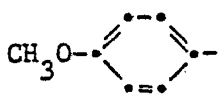 | 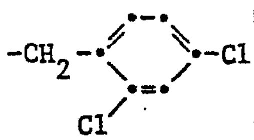 | -J | |
| 4.37 | 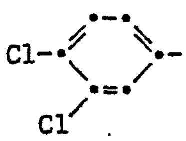 | $-C_3H_7(n)$ | -Cl | |
| 4.38 | 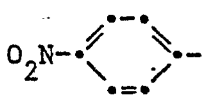 | 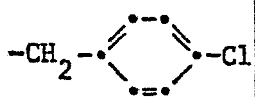 | -Cl | |
| 4.39 | 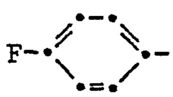 | 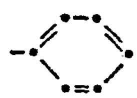 | -Cl | |
| 4.40 | 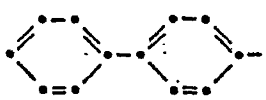 | $-C_3H_7(n)$ | -J | |
| 4.41 | 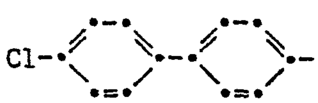 | 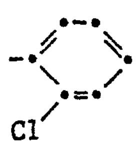 | -Cl | |

Tabelle IV: (Fortsetzung)

| No. | 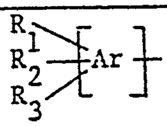 | $R_4$ | A | phys. Konst. |
|---|---|---|---|---|
| 4.42 | 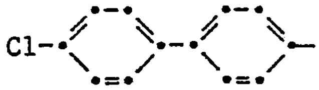 | $-C_4H_9$(t) | -Cl | |
| 4.43 | 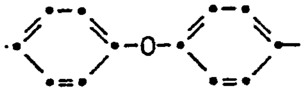 | $-C_3H_7$(n) | -Cl | |
| 4.44 | 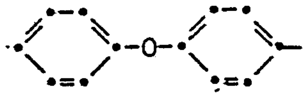 | $-C_3H_7$(n) | $-OSO_2CH_3$ | |
| 4.45 | 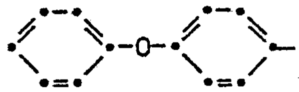 | $-CH_2-CH{=}CH_2$ | -Cl | |
| 4.46 | 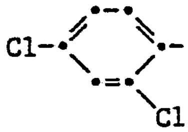 | $-CH_2OCH_3$ | -Cl | |
| 4.47 | 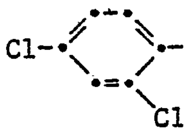 | $-CH_2OCH_3$ | -Br | |
| 4.48 | 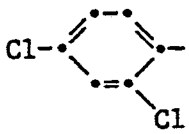 | $-CH_2OC_2H_5$ | $-OSO_2CH_3$ | |
| 4.49 | 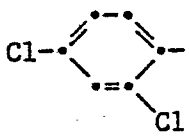 | $-CH_2OC_3H_7$-n | $-OCOCH_3$ | |
| 4.50 | 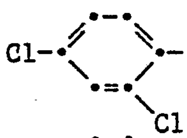 | $-CH_2O-C_6H_5$ | 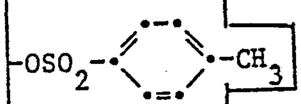 | |
| 4.51 | 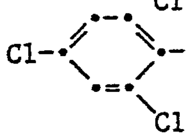 | $-CH_2O-C_6H_5$ | -Cl | |
| 4.52 | 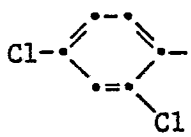 | $-CH_2O-C_6H_4Cl(4)$ | -Cl | Oel |
| 4.53 | 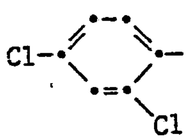 | $-CH(CH_3)_2-CN$ | -Cl | |
| 4.54 | 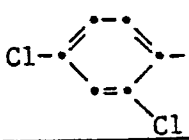 | $-CH_2O-C_6H_3Cl_2(2,4)$ | -J | |

Tabelle IV (Fortsetzung)

| No. | 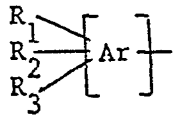 | $R_4$ | A | phys. Konst. |
|---|---|---|---|---|
| 4.55 | 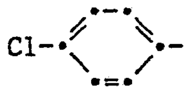 | $-CH_2CH_2OCH_3$ | $-OCOCH_3$ | |
| 4.56 | 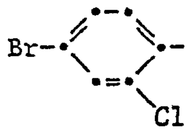 | $-CH_2SCH_3$ | -Cl | |
| 4.57 | 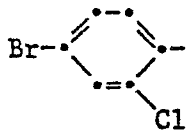 | $-CH_2O-C_6H_4NO_2(4)$ | -Cl | |
| 4.58 | 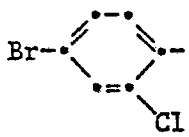 | $-C(CH_3)_2-COOCH_3$ | $-OCOCH_3$ | |
| 4.59 | 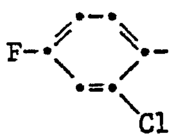 | $-CH_2CH_2O-C_6H_4F(4)$ | -Cl | |
| 4.60 | 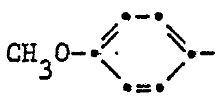 | $-CH_2O-C_6H_5$ | -Cl | |
| 4.61 | 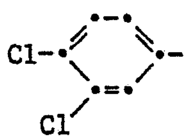 | $-CH_2O-C_6H_4Cl(4)$ | -Cl | |
| 4.62 | 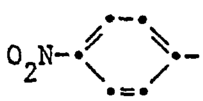 | $-CH_2OCH_3$ | -Cl | |
| 4.63 | 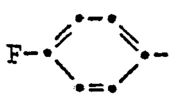 | $-CH_2SCH_3$ | -Cl | |
| 4.64 | 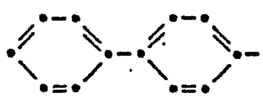 | $-CH_2OCH_3$ | -J | |
| 4.65 | 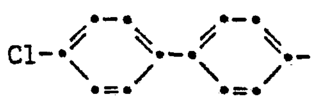 | $-CH_2OCH_3$ | -Cl | |

Tabelle IV (Fortsetzung)

| No. | 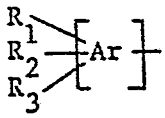 | $R_4$ | A | phys. Konst. |
|---|---|---|---|---|
| 4.66 | 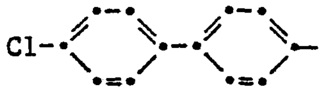 | $-CH_2O-C_6H_5$ | -Cl | |
| 4.67 | 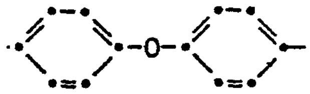 | $-CH_2OCH_3$ | -Cl | |
| 4.68 | 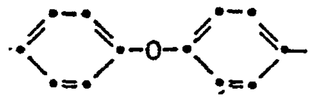 | $-CH_2O-C_6H_4Cl(4)$ | $-OSO_2CH_3$ | |
| 4.69 | 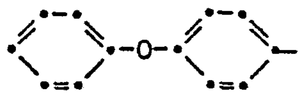 | $-CH_2O-C_6H_3Cl_2(2,4)$ | -Cl | |

Tabelle V: Metallkomplexe der Formel

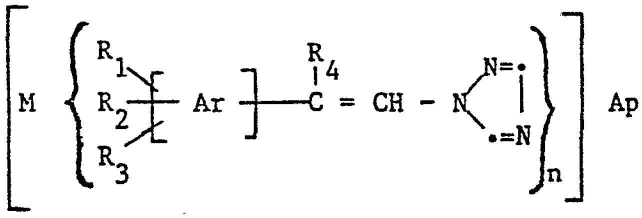

M = Metall

A = Anion einer organischen oder anorganischen Säure

p = 1, 2

n = 1, 2

| Verb. No. | 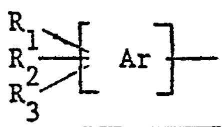 | $R_4$ | M | A | p | n |
|---|---|---|---|---|---|---|
| 5.1 | 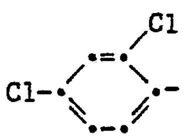 | $C_3H_7$-n | Cu | Cl | 2 | 2 |
| 5.2 | 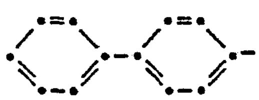 | 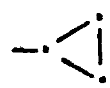 | Cu | Cl | 2 | 2 |
| 5.3 | 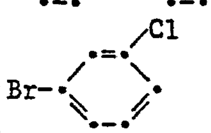 | $C_3H_7$-n | Ni | Cl | 2 | 2 |
| 5.4 | 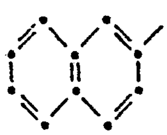 | $C_2H_5$ | Cu | Cl | 2 | 2 |
| 5.5 | 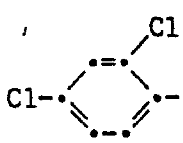 | 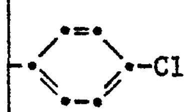 | Zn | Cl | 2 | 2 |
| 5.6 | 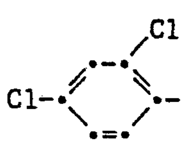 | $C_3H_7$-n | Sn | Cl | 4 | 2 |

Tabelle V: (Fortsetzung)

| Verb. No. | $R_1, R_2, R_3$ [ Ar ] | $R_4$ | M | A | p | n |
|---|---|---|---|---|---|---|
| 5.7 | 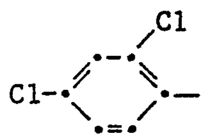 | $C_3H_7$-n | Zr | $OCl_2$ | 1 | 2 |
| 5.8 | 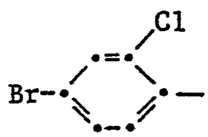 | $C_4H_9$-n | Zr | $OCl_2$ | 1 | 2 |
| 5.9 | 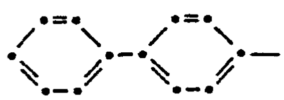 | 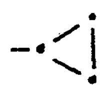 | Zr | $OCl_2$ | 1 | 2 |
| 5.10 | 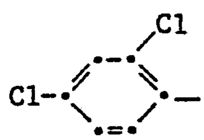 | $C_3H_7$-i | Zr | $OCl_2$ | 1 | 2 |
| 5.11 | 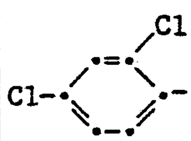 | $-CH_2OCH_3$ | Cu | Cl | 2 | 2 |
| 5.12 | 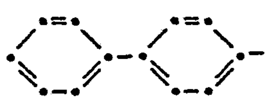 | $-CH_2OCH_3$ | Cu | Cl | 2 | 2 |
| 5.13 | 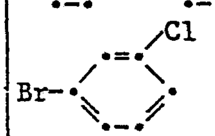 | $-CH_2OC_6H_5$ | Ni | Cl | 2 | 2 |
| 5.14 | 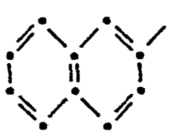 | $-CH_2OC_6H_5$ | Cu | Cl | 2 | 2 |
| 5.15 | 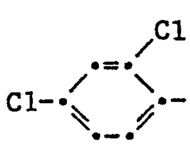 | $-CH_2OC_6H_4Cl(4)$ | Zn | Cl | 2 | 2 |
| 5.16 | 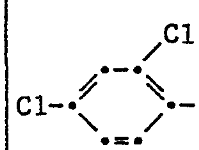 | $-CH_2OC_6H_3Cl_2(2,4)$ | Sn | Cl | 4 | 2 |

Tabelle VI: Quaternäre Azoliumsalze der Formel

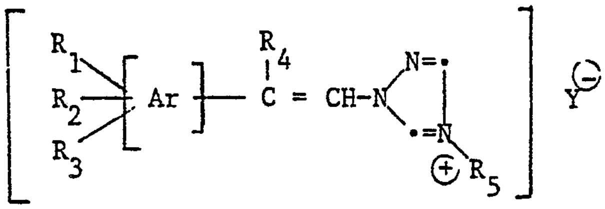

| Verb. No. | 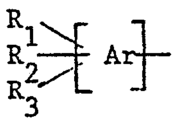 | $R_4$ | $R_5$ | Y |
|---|---|---|---|---|
| 6.1 | 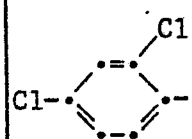 | $C_2H_5$ | $-CH_3$ | J |
| 6.2 | 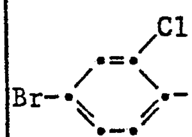 | $C_3H_7$-n | $-C_2H_5$ | J |
| 6.3 | 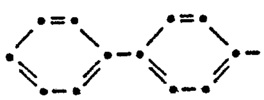 | $C_3H_7$-n | $-CH_2-CH=CH_2$ | Cl |
| 6.4 | 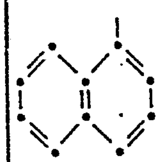 | 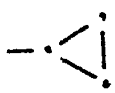 | $-C_4H_9$-n | J |
| 6.5 | 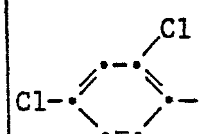 | 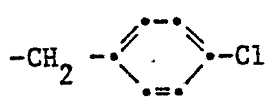 | 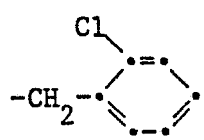 | Cl |
| 6.6 | 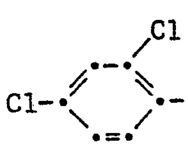 | $-C_3H_7$-n | 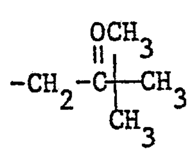 | Br |

Tabelle VI (Fortsetzung)

| Verb. No. | 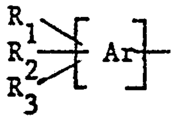 | $R_4$ | $R_5$ | Y |
|---|---|---|---|---|
| 6.7 | 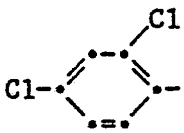 | 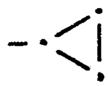 | 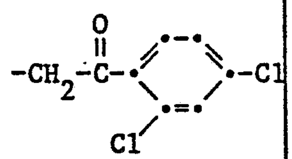 | Br |
| 6.8 | 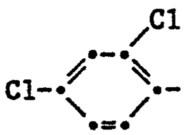 | 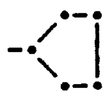 | 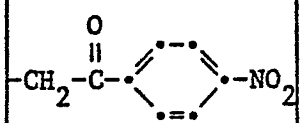 | Br |
| 6.9 | 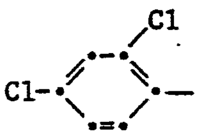 | $C_3H_7$-n | 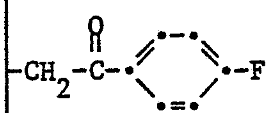 | Br |
| 6.10 | 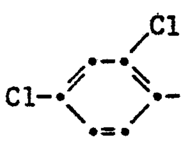 | $C_3H_7$-n | 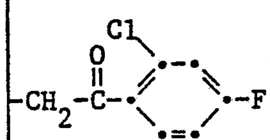 | Br |
| 6.11 | 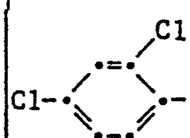 | $-CH_2OCH_3$ | $-CH_3$ | J |
| 6.12 | 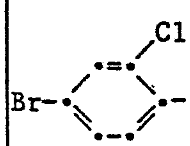 | $-CH_2O-C_6H_5$ | $-C_2H_5$ | J |
| 6.13 | 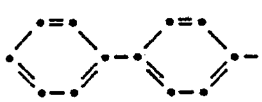 | $-CH_2OCH_2$ | $-CH_2-CH=CH_2$ | Cl |
| 6.14 | 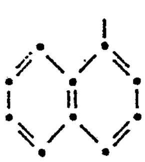 | $-CH_2O-C_6H_4Cl(4)$ | $-C_4H_9$-n | J |

Tabelle VI: (Fortsetzung)

| Verb. No. | 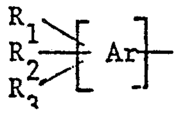 | $R_4$ | $R_5$ | Y |
|---|---|---|---|---|
| 6.15 | 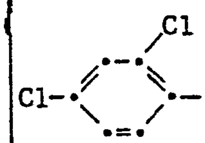 | $-CH_2O-C_6H_3Cl_2(2,4)$ | 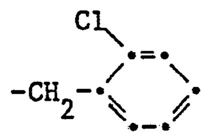 | Cl |
| 6.16 | 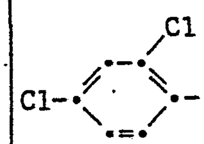 | $-C(CH_3)_2-CN$ | 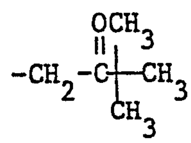 | Br |

Formulierungsbeispiele für flüssige Wirkstoffe der Formel I

(% = Gewichtsprozent)

| 12. Emulsions-Konzentrate | a) | b) | c) |
|---|---|---|---|
| Wirkstoff aus den Tabellen I bis VI | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 13. Lösungen | a) | b) | c) | d) |
|---|---|---|---|---|
| Wirkstoff aus den Tabellen I bis VI | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| 14. Granulate | a) | b) |
|---|---|---|
| Wirkstoff aus den Tabellen I bis VI | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 15. Stäubemittel | a) | b) |
|---|---|---|
| Wirkstoff aus den Tabellen I bis VI | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 16. Spritzpulver | a) | b) | c) |
|---|---|---|---|
| Wirkstoff aus den Tabellen I bis VI | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

17. Emulsions-Konzentrat

| | |
|---|---|
| Wirkstoff aus den Tabellen I bis VI | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 18. Stäubemittel | a) | b) |
|---|---|---|
| Wirkstoff aus den Tabellen I bis VI | 5% | 8% |
| Talkum | 95% | - |
| Kaolin | - | 92% |

Man erhält anwendungsfertige Stäubemittel, indem der Wirkstoff mit den Träger vermischt auf einer geeigneten Mühle vermahlen wird.

19. Extruder Granulat

| | |
|---|---|
| Wirkstoff aus den Tabellen I bis VI | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

20. Umhüllungs-Granulat

| | |
|---|---|
| Wirkstoff aus den Tabellen I bis VI | 3% |
| Polyethylenglykol (M G 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

21. Suspensions-Konzentrat

| | |
|---|---|
| Wirkstoff aus den Tabellen I bis VI | 40% |
| Ethylenglykol | 10% |
| Nonylphenolpolyethylenglykolether (15 Mol Ethylenoxid) | 6% |
| N-Linginsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele:

### Beispiel 22: Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer

Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus den Tabellen I bis VI zeigten gegen Puccinia-Pilze eine gute Wirkung. Unbehandelte aber infizierte Kontrolpflanzen zeigten einen Puccinia-Befall von 100%. Unter anderem hemmten die Verbindungen 1.1a,1.1b,1.5,1.9,1.13,1.25,1.27,1.29,1.31,1.71,1.73,1.75, 1.80,1.101,1.143-1.146,1.154,1.155,1.156,1.161,1.174,2.1-2.4,2.8,3.2, 4.1,4.2,4.4,4.9,4.13,4.17,5.1,5.6,5.10 und 6.2 den Puccinia Befall auf 0 bis 5%.

Beispiel 23: Wirkung gegen Cercospora arachidicola auf Erdnusspflanzen

a) Residual-protektive Wirkung

10 - 15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

b) Systemische Wirkung

Zu 10 - 15 cm hohen Erdnusspflanzen wurde eine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen I bis VI behandelt wurden, einen stark reduzierten Cercospora-Befall. So verhinderten die Verbindungen 1.1a,1.1b,1.5,1.25,1.27,1.29,1.31,1.73,1.75,1.80,1.101,1.144,1.145, 1.146,1.154,1.156,1.161,2.1,2.2,2.4,4.2,2.8,3.2,4.1,4.4,4.9,4.13, 4.17,5.10 und 6.2 in obigen Versuchen das Auftreten von Flecken fast vollständig (0 bis 10%).

Beispiel 24: Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,02% Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die

Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100%. Unter anderen Verbindungen aus den Tabellen I bis VI hemmten Verbindungen Nr. 1.1a,1.1b,1.5,1.9,1.13,1.27,1.29,1.31,1.71, 1.76,1.101,1.143,1.146,1.154,1.155,1.156,1.161,1.174,1.181,2.1-2.4, 3.2,4.1,4.4,5.6 und 6.2 den Pilzbefall auf Gerste auf 0 bis 5%.

Beispiel 25: Residual-protektive Wirkung gegen Venturia inaequalis auf Apfeltrieben.

Apfelstecklinge mit 10 - 20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensupension des Pilzes infiziert. Die Pflanzen wurden dann während 5 Tagen bei 90 - 100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen Nr. 1.1a,1.1b,1.5,1.9,1.13, 1.25,1.29,1.71,1.75,1.76,1.80,1.101,1.143,1.145,2.2,2.3,2.8,3.2,4.1, 4.4,5.1,5.10 und 6.20 hemmten den Krankheitsbefall auf weniger als 10%. Kontrollpflanzen waren 100%ig befallen.

Beispiel 26: Wirkung gegen Botrytis cinerea auf Bohnen

Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der

infizierten Pflanzen während 3 Tagen bei 95-100% relativer Luftfeuchtigkeit und 21°C erfolgte die Beurteilung des Pilzbefalls. Die Verbindungen aus den Tabellen I bis VI hemmten in vielen Fällen die Pilzinfektion sehr stark. Bei einer Konzentration von 0.02% erwiesen sich z.B. die Verbindungen Nr. 1.1a,1.5,1.9,1.25,1.27,1.29,1.71,1.73,1.75,1.80,1.16, 1.101,1.143-1.46,2.1-2.4,2.8,3.2,4.1,4.2,4.4,4.9,5.1,5.6,5.10 und 6.2 als voll wirksam. Der Krankheitsbefall lag bei 0 bis 8%.

Der Botrytis-Befall unbehandelter aber infizierter Bohnenpflanzen betrug 100%.

Patentansprüche (für alle benannten Länder ausser Oesterreich)

1. Verbindungen der allgemeinen Formel I,

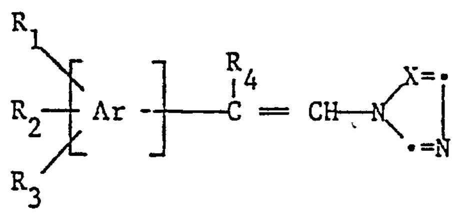

(I)

worin X für -CH= oder -N= steht;

Ar für Phenyl, Biphenyl-4-yl, α-Naphthyl, β-Naphthyl oder 4-Phenoxyphenyl steht;

$R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten;

$R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, Phenyl, 2-Pyridyl, 3-Pyridyl, 4-Pyridyl, 2-Thienyl oder eine durch $C_3$-$C_8$-Cycloalkyl, Phenoxy, Benzyloxy, $C_1$-$C_6$-Alkoxy, -COO-$C_1$-$C_4$-Alkyl, Cyano oder Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;

unter Einschluss der Säureadditionssalze, quaternären Azoliumsalze und Metallkomplexe.

2. Verbindungen der Formel I nach Anspruch 1, worin Ar für Phenyl, Biphenyl-4-yl, α-Naphthyl oder β-Naphthyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; X für -CH= oder -N= steht; und $R_4$ $C_1$-$C_{10}$-Alkyl, $C_3$-$C_8$-Cycloalkyl, Phenyl, $C_2$-$C_5$-Alkenyl, 2-Pyridyl, 3-Pyridyl, 4-Pyridyl, 2-Thienyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor, Brom, Methyl, Methoxy oder Trifluormethyl substituiert ist.

3. Verbindungen der Formel I nach Anspruch 2, worin Ar für Phenyl, Biphenyl-4-yl oder α-Naphthyl steht; $R_1$, $R_2$, $R_3$ unabhängig voneinander für Wasserstoff, Fluor, Chlor, Brom, Nitro, $C_1$-$C_3$-Alkyl, $C_1$-$C_3$-Alkoxy oder Trifluormethyl stehen; X für -N= steht; und $R_4$ $C_1$-$C_6$-Alkyl, $C_3$-$C_6$-Cycloalkyl, Phenyl oder eine durch $C_3$-$C_6$-Cycloalkyl oder durch Phenyl Phenyl substituierte $C_1$-$C_4$-Alkylgruppe bedeutet, worin jeder Phenylanteil unsubstituiert oder durch Fluor, Chlor, Methyl, Methoxy oder Trifluormethyl substituiert ist.

4. Verbindungen der Formel I nach Anspruch 3, worin Ar für Phenyl oder Biphenyl-4-yl steht; $R_1$, $R_2$, $R_3$ unabhängig voneinander Wasserstoff, Fluor, Chlor, Brom, Methyl, Ethyl, Methoxy oder Trifluormethyl bedeutet; X für -N= steht; und $R_4$ $C_1$-$C_4$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor oder Methyl substituiert ist.

5. Verbindungen der Formel I nach Anspruch 1, worin das Molekülfragment

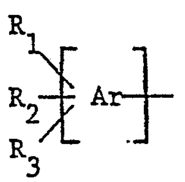

für α-Naphthyl, 2-Halo-α-Naphthyl, 4-Halo-α-naphthyl, β-Naphthyl, Biphenyl-4-yl, 4'-Halobiphenyl-4-yl, 2'-Halobiphenyl-4-yl, 2-Halophenyl, 4-Halophenyl oder 2,4-Dihalophenyl steht; und $R_4$ $C_1$-$C_4$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor oder Methyl substituiert ist.

6. Verbindungen der Formel I nach Anspruch 1, worin Ar für Phenyl steht; $R_1$ Wasserstoff, 2-Fluor, 2-Chlor, 2-Brom, 2-Methyl oder 2-Trifluormethyl; $R_2$ Wasserstoff, 4-Fluor, 4-Chlor, 4-Brom, 4-Methyl oder 4-Trifluormethyl, $R_3$ Wasserstoff, 6-Fluor, 6-Chlor, 6-Brom, 6-Methyl oder 6-Trifluormethyl bedeuten; und $R_4$ für Methyl, Ethyl, n-Propyl,

iso-Propyl, sek.-Propyl, n-Butyl, sek.-Butyl, tert.-Butyl, iso-Butyl, Cyclopropyl, Cyclopentyl, Cyclohexyl, Phenyl, 2-Chlorphenyl oder 2,4-Dichlorphenyl steht.

7. Verbindungen der Formel I nach Anspruch 1, worin das Molekülfragment

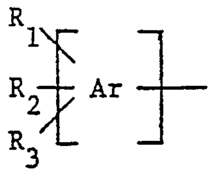

einen gegebenenfalls durch $C_1$-$C_4$-Alkyl, $C_1$-$C_4$-Alkoxy, Cyano, Nitro, Trifluormethyl oder ein bis drei Halogenatome substituierten Phenylrest bedeutet, X für -N= steht und $R_4$ für $C_1$-$C_{10}$-Alkyl, $C_2$-$C_5$-Alkenyl, $C_3$-$C_8$-Cycloalkyl, gegebenenfalls durch $C_3$-$C_8$-Cycloalkyl oder Phenyl substituiertes $C_1$-$C_4$-Alkyl steht, wobei der Phenylsubstituent seinerseits gegebenenfalls durch Halogen, $C_1$-$C_4$-Alkyl oder $C_1$-$C_4$-Alkxoy substituiert sein kann.

8. Verbindungen der Formel I nach Anspruch 1, worin X für -CH= oder -N= steht; Ar für Phenyl, Biphenyl-4-yl, α-Naphthyl, β-Naphthyl oder 4-Phenoxyphenyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; $R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, Phenyl, 2-Pyridyl, 3-Pyridyl, 4-Pyridyl, 2-Thienyl oder eine durch $C_3$-$C_8$-Cycloalkyl oder Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;

unter Einschluss der Säureadditionssalze, quaternären Azoliumsalze und Metallkomplexe.

9. Verbindungen der Formel I nach Anspruch 1, worin X für -CH= oder -N= steht; Ar für Phenyl, Biphenyl-4-yl, α-Naphthyl, β-Naphthyl oder 4-Phenoxyphenyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; $R_4$ eine durch Phenoxy, Benzyloxy, $C_1$-$C_6$-Alkoxy, -COO-$C_1$-$C_4$-Alkyl oder Cyano substituierte $C_1$-$C_{10}$-Alkylgruppe bedeutet, wobei jeder Phenylanteil unsubstituiert oder durch Halogen, $C_1$-$C_4$-Alkyl, Methoxy, Nitro oder Trifluormethyl substituiert ist; unter Einschluss der Säureadditionssalze, quaternären Azoliumsalze und Metallkomplexe.

10. Eine Verbindung ausgewählt aus der Reihe
α-Ethyl-β-(1H-1,2,4-triazol-1'-yl)-2,4-dichlorstyrol,
α-(n)-Propyl-β-(1H-1,2,4-triazol-1'-yl)-2,4-dichlorstyrol,
α-iso-Propyl-β-(1H-1,2,4-traizol-1'-yl)-2,4-dichlorstyrol,
α-(n)-Butyl-β-(1H-1,2,4-triazol-1'-yl)-2,4-dichlorstyrol,
α-(n)-Pentyl-β-(1H-1,2,4-triazol-1'-yl)-2,4-dichlorstyrol,
α-sek.-Butyl-β-(1H-1,2,4-triazol-1'-yl)-2,4-dichlorstyrol,
α-Ethyl-β-(1H-1,2,4-triazol-1'-yl)-2-chlor-4-bromstyrol,
α-(n)-Propyl-β-(1H-1,2,4-triazol-1'-yl)-2-chlor-4-bromstyrol,
α-iso-Propyl-β-(1H-1,2,4-triazol-1'-yl)-2-chlor-4-bromstyrol,
α-(n)-Butyl-β-(1H-1,2,4-triazol-1'-yl)-2-chlor-4-bromstyrol,
α-sek.-Butyl-β-(1H-1,2,4-triazol-1'-yl)-2-chlor-4-bromstyrol,
α-n-Pentyl-β-(1H-1,2,4-triazol-1'-yl)-2-chlor-4-bromstyrol,
α-Cyclopropyl-β-(1H-1,2,4-triazol-1'-yl)-2,4-dichlorstyrol,
α-Cyclopropyl-β-(1H-1,2,4-triazol-1'-yl)-2-chlor-4-bromstyrol,
α-Cyclopentyl-β-(1H-1,2,4-triazol-1'-yl)-2,4-dichlorstyrol,
α-Cyclopentyl-β-(1H-1,2,4-triazol-1'-yl)-2-chlor-4-bromstyrol,
α-(n)-Propyl-β-(1H-1,2,4-triazol-1'-yl)-4-chlorstyrol,
α-(n)-Propyl-β-(1H-1,2,4-triazol-1'-yl)-2-chlorstyrol,
α-Cyclopropyl-β-1H-1,2,4-triazol-1'-yl)-4-chlorstyrol,
α-(n)-Propyl-β-(1H-1,2,4-triazol-1'-yl)-2-chlor-4-fluorstyrol,
α-Cyclohexyl-β-(1H-1,2,4-triazol-1'-yl)-4-chlorstyrol und
α-(n-)-Propyl-β-(1H-imidazol-1'-yl)-2,4-dichlorstyrol.

11. Eine Verbindung ausgewählt aus der Reihe

α-(4-Fluorphenoxymethyl)-β-(1H-1,2,4-triazol-1'-yl)-2,4-dichlor-styrol,
α-(4-tert.Butylphenoxymethyl)-β-(1H-1,2,4-triazol-1'-yl)-2,4-di-chlorstyrol,
α-(4-Chlorphenoxymethyl)-β-(1H-1,2,4-triazol-1'-yl)-2,4-dichlorstyrol,
α-(Phenoxymethyl)-β-(1H-1,2,4-triazol-1'-yl)-2,4-dichlorstyrol und
α-(2,4-Dichlorphenoxymethyl)-β-(1H-1,2,4-triazol-1'-yl)-4-methoxy-styrol.

12. Verfahren zur Herstellung von 1-Aryl-2-azolyl-ethen-derivaten der Formel I nach Anspruch 1, dadurch gekennzeichnet, dass man entweder nach Variante i ein Arylketon der Formel II,

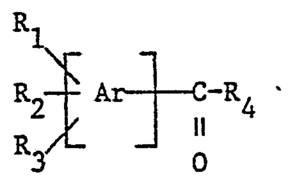

(II)

worin Ar, $R_1$, $R_2$, $R_3$ und $R_4$ die unter Formel I angegebenen Bedeutungen haben, mit einem 1H-Azol-1-yl-methylphosphonat der Formel III,

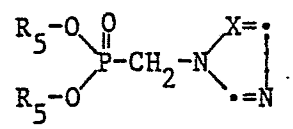

(III)

worin die Reste $R_5$ unabhängig voneinander Phenyl oder $C_1$-$C_4$-Alkyl bedeuten und X für -CH= oder -N= steht, oder mit einem Phosphoniumsalz der Formel,

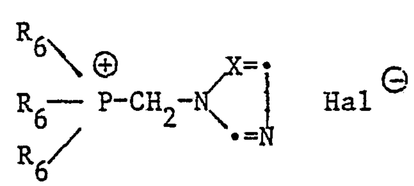

(IV)

worin die Reste $R_6$ unabhängig voneinander Phenyl oder gegebenenfalls durch Hydroxyl substituiertes $C_1$-$C_4$-Alkyl bedeuten, X für -CH= oder -N= steht, und Hal für Chlor oder Brom steht in einem reaktionsinerten Lösungsmittel in Gegenwart einer starken Base umsetzt, oder gemäss Variante ii, indem man direkt aus einer Verbindung der Formel V,

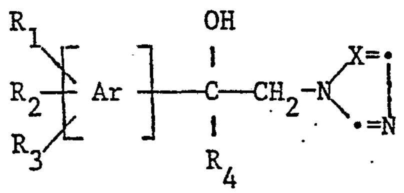

(V)

worin Ar, $R_1$, $R_2$, $R_3$, $R_4$ und X die unter Formel I angegebenen Bedeutungen haben, mit einem wasserentziehenden Mittel Wasser abspaltet, oder indem man die Verbindung der Formel V durch Austausch der freien Hydroxylgruppe gegen eine der üblichen Abgangsgruppen A zuerst in eine Verbindung der Formel VI überführt

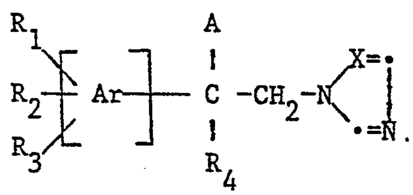

(VI)

und aus VI dann durch Zusatz einer Base die hydrierte Abgangsgruppe H-A abspaltet; oder indem man nach Variante iii ein 1-Aryl-2,2-dichlor-ethen-derivat der Formel VII,

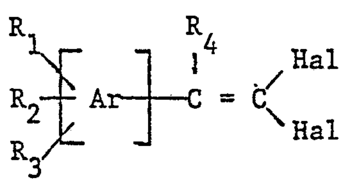

(VII) ,

worin Ar, $R_1$, $R_2$, $R_3$ und $R_4$ die unter Formel I angegebenen Bedeutungen haben, und die Substituenten Hal unabhängig voneinander in der Schmelze bei +150° bis +350°C und gegebenenfalls unter Druck mit überschüssigem Azol der Formel VIII,

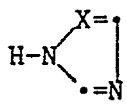

(VIII)

worin X für -CH= oder -N= steht, umsetzt; oder indem man nach Variante iv eine Verbindung der Formel IX,

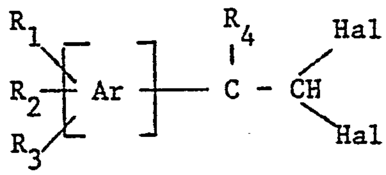

(IX)

worin Ar, $R_1$, $R_2$, $R_3$ und $R_4$ die unter Formel I angegebenen Bedeutungen haben, für Halogen stehen, in einem dipolaren aprotischen Lösungsmittel und gegebenenfalls in Gegenwart einer Base bei +40° bis +180°C oder in der Schmelze bei +150° bis +350°C und gegebenenfalls in Gegenwart einer Base mit überschüssigem Azol der Formel VIII umsetzt.

13. Mikrobizides Mittel zur Bekämpfung und/oder Verhütung eines Befalls von Pflanzen durch Mikroorganismen, dadurch gekennzeichnet, dass es als mindestens eine aktive Komponente ein neues 1-Aryl-2-azolyl-ethenderivat der Formel I gemäss Anspruch 1 enthält.

14. Mittel gemäss Anspruch 13, dadurch gekennzeichnet, dass es als aktive Komponente mindestens eine der in den Ansprüchen 2 bis 11 definierten Verbindungen enthält.

15. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 11 auf die Pflanze oder deren Standort appliziert.

16. Verfahren nach Anspruch 15 von Verbindungen der Formel I gemäss einem der Ansprüche 2 bis 11.

17. Verfahren gemäss einem der Ansprüche 15 oder 16, dadurch gekennzeichnet, dass es sich bei den Mikroorganismen um phytopathogene Pilze handelt.

18. Die Verbindungen der Formel VI,

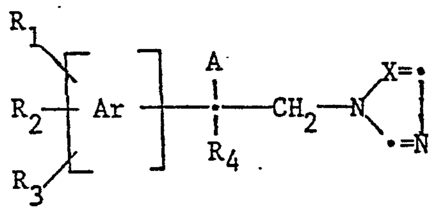

(VI)

worin Ar, $R_1$, $R_2$, $R_3$, $R_4$ und X die unter Formel I in Anspruch 1 angegebenen Bedeutungen haben und der Substituent A für Chlor, Brom, Jod oder eine der Gruppen $-OCO-R_7$ oder $-OSO_2-R_7$ steht, wobei $R_7$ für $C_1$-$C_3$-Alkyl, $C_1$-$C_3$-Haloalkyl oder gegebenenfalls durch Halogen, Methyl, Nitro, Trifluormethyl oder Methoxy substituiertes Phenyl steht.

19. Verbindungen der Formel VI nach Anspruch 18, dadurch gekennzeichnet, dass die Substituenten Ar, $R_1$, $R_2$, $R_3$, $R_4$ und X wie in Formel I unter Anspruch 1 definiert sind und A für Chlor oder Brom steht.

Patentansprüche (für Oesterreich)

1. Mikrobizides Mittel zur Bekämpfung und/oder Verhütung eines Befalls von Pflanzen durch Mikroorganismen, dadurch gekennzeichnet, dass es als mindestens eine aktive Komponente ein neues 1-Aryl-2-azolyl-ethenderivat der Formel I enthält,

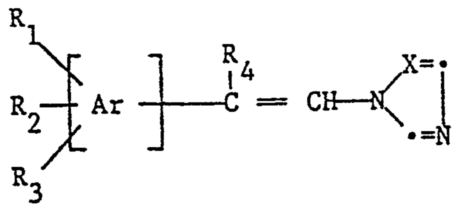

(I)

worin X für -CH= oder -N= steht;

Ar für Phenyl, Biphenyl-4-yl, α-Naphthyl, β-Naphthyl oder 4-Phenoxyphenyl steht;

$R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten;

$R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, Phenyl, 2-Pyridyl, 3-Pyridyl, 4-Pyridyl, 2-Thienyl oder eine durch $C_3$-$C_8$-Cycloalkyl, Phenoxy, Benzyloxy, $C_1$-$C_6$-Alkoxy, -COO-$C_1$-$C_4$-Alkyl, Cyano oder Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;

unter Einschluss der Säureadditionssalze, quaternären Azoliumsalze und Metallkomplexe.

2. Mittel nach Anspruch 1, enthaltend eine der Verbindungen der Formel I, worin Ar für Phenyl, Biphenyl-4-yl, α-Naphthyl oder β-Naphthyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; X für -CH= oder -N= steht; und $R_4$ $C_1$-$C_{10}$-Alkyl, $C_3$-$C_8$-Cycloalkyl, Phenyl, $C_2$-$C_5$-Alkenyl, 2-Pyridyl, 3-Pyridyl,

4-Pyridyl, 2-Thienyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor, Brom, Methyl, Methoxy oder Trifluormethyl substituiert ist.

3. Mittel nach Anspruch 2, enthaltend eine der Verbindungen der Formel I, worin Ar für Phenyl, Biphenyl-4-yl oder α-Naphthyl steht; $R_1$, $R_2$, $R_3$ unabhängig voneinander für Wasserstoff, Fluor, Chlor, Brom, Nitro, $C_1$-$C_3$-Alkyl, $C_1$-$C_3$-Alkoxy oder Trifluormethyl stehen; X für -N= steht; und $R_4$ $C_1$-$C_6$-Alkyl, $C_3$-$C_6$-Cycloalkyl, Phenyl oder eine durch $C_3$-$C_6$-Cycloalkyl oder durch Phenyl Phenyl substituierte $C_1$-$C_4$-Alkylgruppe bedeutet, worin jeder Phenylanteil unsubstituiert oder durch Fluor, Chlor, Methyl, Methoxy oder Trifluormethyl substituiert ist.

4. Mittel nach Anspruch 3, enthaltend eine der Verbindungen der Formel I, worin Ar für Phenyl oder Biphenyl-4-yl steht; $R_1$, $R_2$, $R_3$ unabhängig voneinander Wasserstoff, Fluor, Chlor, Brom, Methyl, Ethyl, Methoxy oder Trifluormethyl bedeutet; X für -N= steht; und $R_4$ $C_1$-$C_4$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor oder Methyl substituiert ist.

5. Mittel nach Anspruch 1, enthaltend eine der Verbindungen der Formel I, worin das Molekülfragment

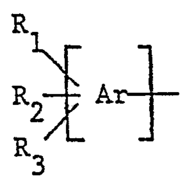

für α-Naphthyl, 2-Halo-α-Naphthyl, 4-Halo-α-naphthyl, β-Naphthyl, Biphenyl-4-yl, 4'-Halobiphenyl-4-yl, 2'-Halobiphenyl-4-yl, 2-Halophenyl, 4-Halophenyl oder 2,4-Dihalophenyl steht; und $R_4$ $C_1$-$C_4$-Alkyl, $C_3$-$C_6$-Cycloalkyl, Phenyl oder eine durch $C_3$-$C_6$-Cycloalkyl oder durch Phenyl substituierte $C_1$-$C_4$-Alkylgruppe bedeutet, worin jeder Phenylanteil unsubstituiert oder durch Fluor, Chlor oder Methyl substituiert ist.

6. Mittel nach Anspruch 1, enthaltend eine der Verbindungen der Formel I, worin Ar für Phenyl steht; $R_1$ Wasserstoff, 2-Fluor, 2-Chlor, 2-Brom, 2-Methyl oder 2-Trifluormethyl; $R_2$ Wasserstoff, 4-Fluor, 4-Chlor, 4-Brom, 4-Methyl oder 4-Trifluormethyl, $R_3$ Wasserstoff, 6-Fluor, 6-Chlor, 6-Brom, 6-Methyl oder 6-Trifluormethyl bedeuten; und $R_4$ für Methyl, Ethyl, n-Propyl, iso-Propyl, sek.-Propyl, n-Butyl, sek.-Butyl, tert.-Butyl, iso-Butyl, Cyclopropyl, Cyclopentyl, Cyclohexyl, Phenyl, 2-Chlorphenyl oder 2,4-Dichlorphenyl steht.

7. Mittel nach Anspruch 1, enthaltend eine der Verbindungen der Formel I, worin das Molekülfragment

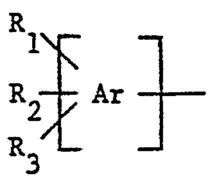

einen gegebenenfalls durch $C_1$-$C_4$-Alkyl, $C_1$-$C_4$-Alkoxy, Cyano, Nitro, Trifluormethyl oder ein bis drei Halogenatome substituierten Phenylrest bedeutet, X für -N= steht und $R_4$ für $C_1$-$C_{10}$-Alkyl, $C_2$-$C_5$-Alkenyl, $C_3$-$C_8$-Cycloalkyl, gegebenenfalls durch $C_3$-$C_8$-Cycloalkyl oder Phenyl substituiertes $C_1$-$C_4$-Alkyl steht, wobei der Phenylsubstituent seinerseits gegebenenfalls durch Halogen, $C_1$-$C_4$-Alkyl oder $C_1$-$C_4$-Alkxoy substituiert sein kann.

8. Mittel nach Anspruch 1, enthaltend eine der Verbindungen der Formel I, worin X für -CH= oder -N= steht; Ar für Phenyl, Biphenyl-4-yl, α-Naphthyl, β-Naphthyl oder 4-Phenoxyphenyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; $R_4$ in den Fällen, in denen Ar für Phenyl steht, die Bedeutungen $C_2$-$C_5$-Alkenyl, $C_3$-$C_8$-Cycloalkyl, $C_1$-$C_{10}$-Alkyl, Phenyl, 2-Pyridyl, 3-Pyridyl, 4-Pyridyl, 2-Thienyl oder eine durch $C_3$-$C_8$-Cycloalkyl oder Phenyl substituierte $C_1$-$C_{10}$-Alkylgruppe hat, wobei jeder Phenylanteil unsubstituiert oder durch Halogen, $C_1$-$C_4$-Alkyl, Methoxy, Nitro oder Trifluormethyl

substituiert ist und $R_4$ in den Fällen, in denen Ar für Biphenyl-4-yl, α-Naphthyl, β-Naphthyl oder 4-Phenoxyphenyl steht, zusätzlich Wasserstoff bedeutet;
unter Einschluss der Säureadditionssalze, quaternären Azoliumsalze und Metallkomplexe.

9. Mittel nach Anspruch 1, enthaltend eine der Verbindungen der Formel I, worin X für -CH= oder -N= steht; Ar für Phenyl, Biphenyl-4-yl, α-Naphthyl, β-Naphthyl oder 4-Phenoxyphenyl steht; $R_1$, $R_2$, $R_3$ unabhängig voneinander Wasserstoff, Halogen, Cyano, Nitro, $C_1$-$C_4$-Alkyl, $C_1$-$C_4$-Alkoxy oder $C_1$-$C_3$-Haloalkyl bedeuten; $R_4$ eine durch Phenoxy, Benzyloxy, $C_1$-$C_6$-Alkoxy, -COO-$C_1$-$C_4$-Alkyl oder Cyano substituierte $C_1$-$C_{10}$-Alkylgruppe bedeutet, wobei jeder Phenylanteil unsubstituiert oder durch Halogen, $C_1$-$C_4$-Alkyl, Methoxy, Nitro oder Trifluormethyl substituiert ist; unter Einschluss der Säureadditionssalze, quaternären Azoliumsalze und Metallkomplexe.

10. Mittel nach Anspruch 1, enthaltend eine Verbindung ausgewählt aus der Reihe

α-Ethyl-β-(1H-1,2,4-triazol-1'-yl)-2,4-dichlorstyrol,
α-(n)-Propyl-β-(1H-1,2,4-triazol-1'-yl)-2,4-dichlorstyrol,
α-iso-Propyl-β-(1H-1,2,4-traizol-1'-yl)-2,4-dichlorstyrol,
α-(n)-Butyl-β-(1H-1,2,4-triazol-1'-yl)-2,4-dichlorstyrol,
α-(n)-Pentyl-β-(1H-1,2,4-triazol-1'-yl)-2,4-dichlorstyrol,
α-sek.-Butyl-β-(1H-1,2,4-triazol-1'-yl)-2,4-dichlorstyrol,
α-Ethyl-β-(1H-1,2,4-triazol-1'-yl)-2-chlor-4-bromstyrol,
α-(n)-Propyl-β-(1H-1,2,4-triazol-1'-yl)-2-chlor-4-bromstyrol,
α-iso-Propyl-β-(1H-1,2,4-triazol-1'-yl)-2-chlor-4-bromstyrol,
α-(n)-Butyl-β-(1H-1,2,4-triazol-1'-yl)-2-chlor-4-bromstyrol,
α-sek.-Butyl-β-(1H-1,2,4-triazol-1'-yl)-2-chlor-4-bromstyrol,
α-n-Pentyl-β-(1H-1,2,4-triazol-1'-yl)-2-chlor-4-bromstyrol,
α-Cyclopropyl-β-(1H-1,2,4-triazol-1'-yl)-2,4-dichlorstyrol,
α-Cyclopropyl-β-(1H-1,2,4-triazol-1'-yl)-2-chlor-4-bromstyrol,
α-Cyclopentyl-β-(1H-1,2,4-triazol-1'-yl)-2,4-dichlorstyrol,

α-Cyclopentyl-β-(1H-1,2,4-triazol-1'-yl)-2-chlor-4-bromstyrol,
α-(n)-Propyl-β-(1H-1,2,4-triazol-1'-yl)-4-chlorstyrol,
α-(n)-Propyl-β-(1H-1,2,4-triazol-1'-yl)-2-chlorstyrol,
α-Cyclopropyl-β-1H-1,2,4-triazol-1'-yl)-4-chlorstyrol,
α-(n)-Propyl-β-(1H-1,2,4-triazol-1'-yl)-2-chlor-4-fluorstyrol,
α-Cyclohexyl-β-(1H-1,2,4-triazol-1'-yl)-4-chlorstyrol und
α-(n-)-Propyl-β-(1H-imidazol-1'-yl)-2,4-dichlorstyrol.

11. Mittel nach Anspruch 1, enthaltend eine Verbindung ausgewählt aus der Reihe
α-(4-Fluorphenoxymethyl)-β-(1H-1,2,4-triazol-1'-yl)-2,4-dichlorstyrol,
α-(4-tert.Butylphenoxymethyl)-β-(1H-1,2,4-triazol-1'-yl)-2,4-dichlorstyrol,
α-(4-Chlorphenoxymethyl)-β-(1H-1,2,4-triazol-1'-yl)-2,4-dichlorstyrol,
α-(Phenoxymethyl)-β-(1H-1,2,4-triazol-1'-yl)-2,4-dichlorstyrol und
α-(2,4-Dichlorphenoxymethyl)-β-(1H-1,2,4-triazol-1'-yl)-4-methoxystyrol.

12. Verfahren zur Herstellung von 1-Aryl-2-azolyl-ethen-derivaten der Formel I nach Anspruch 1, dadurch gekennzeichnet, dass man entweder nach Variante i ein Arylketon der Formel II,

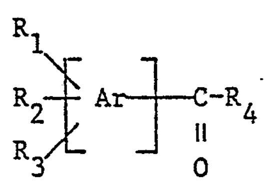

(II)

worin Ar, $R_1$, $R_2$, $R_3$ und $R_4$ die unter Formel I angegebenen Bedeutungen haben, mit einem 1H-Azol-1-yl-methylphosphonat der Formel III,

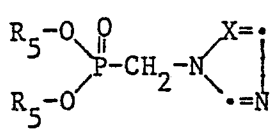

(III)

worin die Reste $R_5$ unabhängig voneinander Phenyl oder $C_1$-$C_4$-Alkyl bedeuten und X für -CH= oder -N= steht, oder mit einem Phosphoniumsalz der Formel,

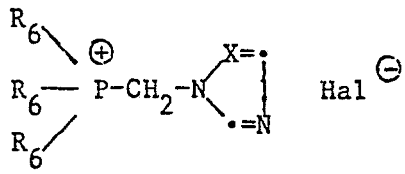

(IV)

worin die Reste $R_6$ unabhängig voneinander Phenyl oder gegebenenfalls durch Hydroxyl substituiertes $C_1$-$C_4$-Alkyl bedeuten, X für -CH= oder -N= steht, und Hal für Chlor oder Brom steht in einem reaktionsinerten Lösungsmittel in Gegenwart einer starken Base umsetzt, oder gemäss Variante ii, indem man direkt aus einer Verbindung der Formel V,

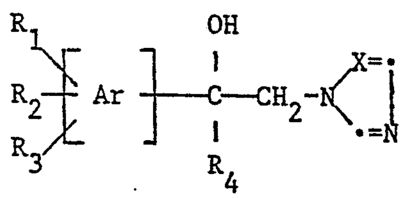

(V)

worin Ar, $R_1$, $R_2$, $R_3$, $R_4$ und X die unter Formel I angegebenen Bedeutungen haben, mit einem wasserentziehenden Mittel Wasser abspaltet, oder indem man die Verbindung der Formel V durch Austausch der freien Hydroxylgruppe gegen eine der üblichen Abgangsgruppen A zuerst in eine Verbindung der Formel VI überführt

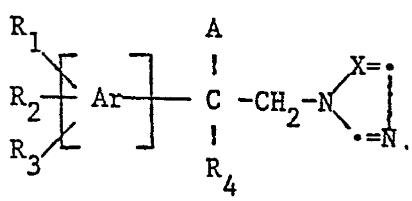

(VI)

und aus VI dann durch Zusatz einer Base die hydrierte Abgangsgruppe H-A abspaltet; oder indem man nach Variante iii ein 1-Aryl-2,2-dichlor-ethen-derivat der Formel VII,

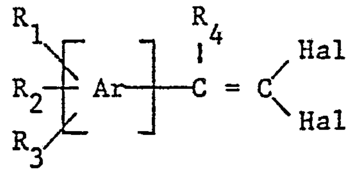

(VII) ,

worin Ar, $R_1$, $R_2$, $R_3$ und $R_4$ die unter Formel I angegebenen Bedeutungen haben, und die Substituenten Hal unabhängig voneinander in der Schmelze bei +150° bis +350°C und gegebenenfalls unter Druck mit überschüssigem Azol der Formel VIII,

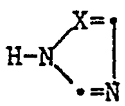

(VIII)

worin X für -CH= oder -N= steht, umsetzt; oder indem man nach Variante iv eine Verbindung der Formel IX,

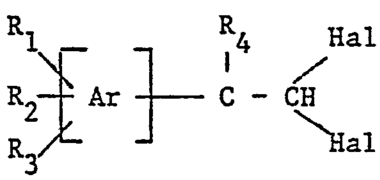

(IX)

worin Ar, $R_1$, $R_2$, $R_3$ und $R_4$ die unter Formel I angegebenen Bedeutungen haben, für Halogen stehen, in einem dipolaren aprotischen Lösungsmittel und gegebenenfalls in Gegenwart einer Base bei +40° bis +180°C oder in der Schmelze bei +150° bis +350°C und gegebenenfalls in Gegenwart einer Base mit überschüssigem Azol der Formel VIII umsetzt.

12. Verfahren zur Bekämpfung oder Verhütung eines Befalls von Kulturpflanzen durch phytopathogene Pilze, dadurch gekennzeichnet, dass man eine der in den Ansprüchen 1 bis 11 definierten Verbindungen der Formel I auf die Pflanze oder deren Standort appliziert.

13. Mikrobizides Mittel zur Bekämpfung und/oder Verhütung eines Befalls von Pflanzen durch Mikroorganismen, dadurch gekennzeichnet, dass es als mindestens eine aktive Komponente eine der Verbindungen der Formel VI

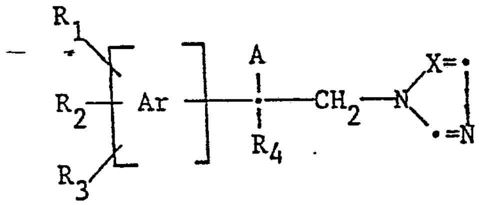

(VI)

enthält, worin Ar, $R_1$, $R_2$, $R_3$, $R_4$ und X die unter Formel I in Anspruch 1 angegebenen Bedeutungen haben und der Substituent A für Chlor, Brom, Jod oder eine der Gruppen $-OCO-R_7$ oder $-OSO_2-R_7$ steht, wobei $R_7$ für $C_1$-$C_3$-Alkyl, $C_1$-$C_3$-Haloalkyl oder gegebenenfalls durch Halogen, Methyl, Nitro, Trifluormethyl oder Methoxy substituiertes Phenyl steht.

14. Mittel nach Anspruch 13, enthaltend eine der Verbindungen der Formel VI nach Anspruch 18, dadurch gekennzeichnet, dass die Substituenten Ar, $R_1$, $R_2$, $R_3$, $R_4$ und X wie in Formel I unter Anspruch 1 definiert sind und A für Chlor oder Brom steht.